# EUROPEAN PATENT APPLICATION

(11) **EP 2 921 502 A1**
(43) Date of publication of application: **23.09.2015**
(21) Application number: 13848052.0
(22) Date of filing: 18.10.2013
(51) Int. Cl.: C07K 16/12, A61K 39/395, A61P 1/00, A61P 1/12, A61P 39/02, C12N 1/15, C12N 1/19, C12N 1/21, C12N 5/10, C12N 15/09, C12P 21/08

(54) **HUMAN ANTIBODY SPECIFIC TO TOXIN PRODUCED FROM CLOSTRIDIUM DIFFICILE, OR ANTIGEN-BINDING FRAGMENT THEREOF**

(30) Priority: 19.10.2012 JP 2012232323
(71) Applicant: Evec Inc., Sapporo-shi Hokkaido 0600042 (JP)
(72) Inventor: TAKADA, Kenzo, Sapporo-shi Hokkaido 060-0042 (JP); WATANABE, Masahiro, Sapporo-shi Hokkaido 060-0042 (JP); TORASHIMA, Takashi, Sapporo-shi Hokkaido 060-0042 (JP)
(74) Representative: EIP
(86) International application number: PCT/JP2013/078326
(87) International publication number: WO 2014/061783

(57) **Abstract**

The present invention provides novel human-derived monoclonal antibodies specifically binding to toxins produced by *Clostridium difficile* (toxin A and toxin B), respectively, and having excellent neutralizing activity, and antigen-binding fragments thereof. The present invention also provides a pharmaceutical composition for the treatment of *Clostridium difficile* infection comprising any of the antibodies or antigen-binding fragments thereof.

## Description

### TECHNICAL FIELD

The present invention relates to human-derived monoclonal antibodies specifically binding to toxins produced by *Clostridium difficile* (toxin A and toxin B), respectively, and antigen-binding fragments thereof, and a pharmaceutical composition for the treatment of *Clostridium difficile* infection comprising any of the antibodies or antigen-binding fragments thereof.

### BACKGROUND ART

In recent years, *Clostridium difficile* infection (hereinafter, abbreviated to CDI) derived from *Clostridium difficile* (hereinafter, referred to as C. *difficile)* has occurred frequently in hospitals, senior-citizen housing facilities, etc., in Europe and the United States. *C. difficile* has received attention as a pathogen responsible for hospital-acquired infection, as with MRSA (methicillin-resistant *Staphylococcus atreus*) and the like. The 2012 report (http://www.cdc.gov/vitalsigns/hai/) of the U.S. Centers for Disease Control and Prevention (CDC) estimates a yearly medical cost of a billion dollars for CDI in the United States and states that the number of yearly deaths in CDI reaches 14,000 people. In 2008, a medical cost (estimate) per patient was as very high as 2,871 to 4,846 dollars for first-time cases and 13,655 to 18,067 dollars for recurrent cases (Non Patent Literature 1).

*C. difficile,* a bacterium belonging to the genus *Clostridium,* is an obligate anaerobe that cannot grow in the presence of oxygen at a concentration of atmospheric level and forms spores. Other bacteria belonging to the genus *Clostridium* include highly pathogenic bacterial species such as *Clostridium tetani* (tetanus bacillus) and *Clostridium botulinum.* In the bacterial name *Clostridium difficile,* the term *"difficile"* is derived from difficulty to separate and culture because *C. difficile* is an obligate anaerobe. *C. difficile* has properties such as unresponsiveness to alcohol disinfection and unresponsiveness to many broad-spectrum antibiotics, and further, a cause of pseudomembranous colitis induced by antibiotics, and these features are considered to be partly responsible for the spread of hospital-acquired *C. difficile* infection (Non Patent Literatures 2, and 3).

CDI is found in every age group, but has a high incidence, particularly, in senior citizens and immune-compromised people. This is probably because broad-spectrum antibiotics are highly frequently used for most of these patients in order to treat various infections; thus, *C. difficile* resistant to these antibiotics grows as a result of microbial substitution in the intestine to produce toxins such as toxin A and toxin B, inducing symptoms such as diarrhea. It has been reported that: patients infected by *C. difficile* have a low antibody titer of an anti-toxin A antibody in serum and develop diarrhea with high frequency (Non Patent Literature 4): and, as for recurrence, the frequency of recurrence is high when the antibody titer of an anti-toxin A antibody in serum is low (Non Patent Literature 5), suggesting that antibodies against *C. difficile* toxins play an important role in protecting against CDI.

The toxins produced by *C. difficile* are mainly toxin A and toxin B. Genes of these toxins are called tcdA and tcdB, respectively, and their gene products are called toxin A (or TcdA) and toxin B (TcdB), respectively. *C. difficile* strains are also classified on the basis of the productivity of these toxins. For example, a toxin A-positive/toxin B-positive strain, a toxin A-negative/toxin B-positive strain, and a toxin A-negative/toxin B-negative strain have been reported. Among them, the toxin A-positive/toxin B-positive strain and the toxin A-negative/toxin B-positive strain are known to cause diarrhea or enteritis (Non Patent Literatures 6 and 7). Recently, an increased number of a highly toxic bacterial strain called *Clostridium difficile* BI/NAP1/027 (PCR ribotype 027/ST1; hereinafter, to as type 027) has been detected in many medical facilities in the United States, Canada, Europe, etc., and is also responsible for the outbreak of CDI. Since the type 027 bacterial strain produces a larger amount of toxin A or toxin B, patients infected by this strain are found more severe. In Japan as well, it was reported in 2005 that type 027 C. *difficile* was separated from the feces of a 30-year-old female patient with pseudomembranous colitis. A.S. Walker et al. in investigation conducted on CDI patients in the U.K. from Sep. 2006 to May 2011 have further revealed the presence of PCR ribotype 078/ST11 (hereinafter, referred to as type 078), which is more lethal than type 027. In addition, this report indicates that patients infected by type 078 appear with a frequency as high as approximately 1/10 of people infected by type 027 (Non Patent Literature 8), suggesting that the need to develop a therapy of CDI has been increasingly urgent.

Most of CDI cases occur highly frequently during the long-term use of antibiotics, and 20 to 30% of diarrhea associated with the administration of antibiotics is reportedly attributed to C. *difficile.* CDI may cause pseudomembranous colitis, which forms a pseudomembrane in the digestive tract, and may remain merely at a mild diarrheal episode or may become severe to cause intestinal obstruction, perforation of the digestive tract, or sepsis, leading to death. Some CDI patients may experience repeated recurrence. Such recurrence may be attributed to the same bacterial strain as the initial C. *difficile* strain or may be attributed to a strain different therefrom. In HIV-infected individuals, C. *difficile* also serves as a primary pathogen that causes bacterial diarrhea.

Therapeutic drugs effective for CDI are limited to metronidazole and vancomycin. Vancomycin, however, might induce the vancomycin resistance of C. *difficile* in the intestine, while metronidazole has neurotoxicity. In consideration of these, it is preferred to avoid using repetitively or for a long period metronidazole or vancomycin at the time of recurrence.

Against this backdrop, many studies are underway toward the early establishment of a further therapy effective for CDI. In this respect, there have been reports on, for example, Ramoplanin (Non Patent Literature 9) and fidaxomicin (Non Patent Literature 10) as antibiotics effective for C. *difficile* infection, and a report on, for example, the maintenance or reconstruction of bacterial flora in the intestine by a yeast *Saccharomyces boulardii* (Non Patent Literature 2) as probiotics (which is a term introduced in contrast to antibiotics and refers to microbes having favorable influence on human bodies, as with, for example, certain kinds of lactic acid bacteria, or products or food products comprising the microbes), and further a report on a toxoid vaccine of toxin A and toxin B deactivated by formalin (Patent Literature 1). Also, there have been reports on a polyclonal antibody against toxin A and toxin B (Patent Literature 2), and monoclonal antibodies against these toxins (Patent Literatures 3, 4, and 5 and Non Patent Literatures 11 and 12). Particularly, monoclonal antibodies 3D8 (also called CDA1) (Patent Literature 3 and Non Patent Literature 12) and MDX1388 (also called 124-152 or CDB1) (Patent Literature 3 and Non Patent Literature 12) against toxin A and toxin B, respectively, joint-developed by the University of Massachusetts and Medarex, Inc. are currently under clinical trial (MK-3415A) for their combined use by Merck KGaA (Non Patent Literature 11).

### CITATION LIST

### PATENT LITERATURE

Patent Literature 1: WO2005/058353
Patent Literature 2: WO2010/094970
Patent Literature 3: WO2006/121422
Patent Literature 4: WO2006/071877
Patent Literature 5: WO2011/130650

### NON PATENT LITERATURE

Non Patent Literature 1: J. Hospital. Infect., 2010, (vol. 74) p. 309
Non Patent Literature 2: Guide to the Elimination of Clostridium difficile in Healthcare Settings (2008)
Non Patent Literature 3: J. Med. Microbiol., 2005, (vol. 54), p. 101
Non Patent Literature 4: N. Engl. J. Med., 2000, (vol. 342) p. 390
Non Patent Literature 5: Lancet. 2001 (357) p. 189
Non Patent Literature 6: J. Med. Microbiol., 2005, (vol. 54), p. 113
Non Patent Literature 7: Clin. Microbial. Rev., 2005, (vol. 18) p. 247
Non Patent Literature 8: Clin. Infect. Dis., 2013, (vol. 56) p. 1589
Non Patent Literature 9: J. Antimicro. Chemo., 2003, (vol. 51), Suppl. S3, iii31.iii35
Non Patent Literature 10: N. Engl. J. Med., 2011 (vol. 364) p. 422
Non Patent Literature 11: N. Engl. J. Med., 2010 (vol. 362) p. 197
Non Patent Literature 12: Inf. Immunity 2006, (vol. 74) p. 6339

### SUMMARY OF INVENTION

### TECHNICAL PROBLEM

CDI is known to bring about a serious gastrointestinal disease in many cases as a result of growth of the bacterium in the digestive tract due to microbial substitution in the intestine caused by antibiotic treatment. In recent years, the spread of hospital-acquired infection by a strongly toxic bacterial strain such as *Clostridium difficile* BI/NAP1/027 and a large number of deaths in this infection have been reported, particularly, in Europe and the United States and have become urgent problems. Under these circumstances, monoclonal antibodies (3D8 and MDX1388) against toxin A and toxin B, respectively, are under clinical research on their combination therapy. Both of these antibodies are still susceptible to improvement in light of their affinity, neutralizing activity, and clinical trials results. For information, results of phase II clinical trial conducted on approximately 200 patients showed significant effects on the rate of recurrence of CDI in an antibody administration group compared with a placebo group, but no significant difference between these groups in terms of the incidence and duration of serious diarrheal episodes, the length of hospitalization of patients, a death rate, etc. (Non Patent Literature 11).

A great majority of antibody drugs approved as medicines are chimeric or humanized antibodies having problems associated with immunogenicity. In nature, completely human-derived antibodies are desirable as antibody drugs. In addition, even the completely human antibody drugs are required to provide pharmaceutical compositions having high neutralizing ability and remarkable therapeutic effects.

### SOLUTION TO PROBLEM

Under these circumstances, the present inventors have conducted diligent studies with the aim of preparing human-derived monoclonal antibodies that exhibit high effectiveness. As a result, the present inventors have successfully prepared an anti-toxin A antibody that recognizes an epitope different from that for 3D8 and hPA-50 (Patent Literature 5) and is characterized in very high affinity and neutralizing activity, and have also successfully prepared an anti-toxin B antibody that recognizes an epitope different from that for MDX1388, and hPA-41 (Patent Literature 5) and has high neutralizing activity. The present inventors have further confirmed *in vivo* that the combined administration of these human antibodies remarkably protects in a dose-dependent manner against lethal action associated with *C. difficile* infection. In this way, the present invention has been completed.

Specifically, the present invention relates to, as described below, a monoclonal antibody specific for toxin A or an antigen-binding fragment thereof, a nucleic acid (polynucleotide) encoding the antibody or the antigen-binding fragment thereof, a vector containing the nucleic acid, and a host cell containing the vector. The present invention also relates to a monoclonal antibody specific for toxin B or a binding fragment thereof, a nucleic acid (polynucleotide) encoding the antibody or the antigen-binding fragment thereof, a vector containing the nucleic acid, and a host cell containing the vector. The present invention further relates to a pharmaceutical composition comprising the antibody of the present invention or the antigen-binding fragment.
[1] An antibody capable of specifically binding to *Clostridium difficile* toxin A protein and neutralizing its biological activity, or an antigen-binding fragment thereof, the antibody containing:
   (i)
      (a) heavy chain CDR1 comprising the amino acid sequence of SEQ ID NO: 4 or an amino acid sequence deviated from the amino acid sequence of SEQ ID NO: 4 by deletion, substitution, insertion, and/or addition mutations of one to several amino acid residues,
      (b) heavy chain CDR2 comprising the amino acid sequence of SEQ ID NO: 5 or an amino acid sequence deviated from the amino acid sequence of SEQ ID NO: 5 by deletion, substitution, insertion, and/or addition mutations of one to several amino acid residues, and
      (c) heavy chain CDR3 comprising the amino acid sequence of SEQ ID NO: 6 or an amino acid sequence deviated from the amino acid sequence of SEQ ID NO: 6 by deletion, substitution, insertion, and/or addition mutations of one to several amino acid residues; and
   (ii)
      (a) light chain CDR1 comprising the amino acid sequence of SEQ ID NO: 10 or an amino acid sequence deviated from the amino acid sequence of SEQ ID NO: 10 by deletion, substitution, insertion, and/or addition mutations of one to several amino acid residues,
      (b) light chain CDR2 comprising the amino acid sequence of SEQ ID NO: 11 or an amino acid sequence deviated from the amino acid sequence of SEQ ID NO: 11 by deletion, substitution, insertion, and/or addition mutations of one to several amino acid residues, and
      (c) light chain CDR3 comprising the amino acid sequence of SEQ ID NO: 12 or an amino acid sequence deviated from the amino acid sequence of SEQ ID NO: 12 by deletion, substitution, insertion, and/or addition mutations of one to several amino acid residues.
[2] The antibody or an antigen-binding fragment thereof according to [1], comprising:
   (i)
      (a) the amino acid sequence of heavy chain CDR1 comprising the amino acid sequence of SEQ ID NO: 4,
      (b) the amino acid sequence of heavy chain CDR2 comprising the amino acid sequence of SEQ ID NO: 5, and
      (c) the amino acid sequence of heavy chain CDR3 comprising the amino acid sequence of SEQ ID NO: 6; and
   (ii)
      (a) the amino acid sequence of light chain CDR1 comprising the amino acid sequence of SEQ ID NO: 10,
      (b) the amino acid sequence of light chain CDR2 comprising the amino acid sequence of SEQ ID NO: 11, and
      (c) the amino acid sequence of light chain CDR3 comprising the amino acid sequence of SEQ ID NO: 12.
[3] The antibody or the antigen-binding fragment thereof according to [1] or [2], comprising
   (a) a heavy chain variable region (VH) comprising the amino acid sequence of SEQ ID NO: 3 or an amino acid sequence having 95% or higher identity to the amino acid sequence of SEQ ID NO: 3, and
   (b) a light chain variable region (VL) comprising the amino acid sequence of SEQ ID NO: 9 or an amino acid sequence having 95% or higher identity to the amino acid sequence of SEQ ID NO: 9.
[4] The antibody or an antigen-binding fragment thereof according to any one of [1] to [3], comprising
   (a) a heavy chain variable region (VH) comprising the amino acid sequence of SEQ ID NO: 3, and
   (b) a light chain variable region (VL) comprising the amino acid sequence of SEQ ID NO: 9.
[5] The antibody or an antigen-binding fragment thereof according to any one of [1] to [4], wherein the antibody or the antigen-binding fragment thereof is of IgG1 (κ) class (subclass).
[6] The antibody or an antigen-binding fragment thereof according to any one of [1] to [5], wherein the antibody or the antigen-binding fragment thereof has a dissociation constant (K_{D} value) of 1 x 10⁻⁹ M or lower against the *Clostridium difficile* toxin A.
[7] The antibody or an antigen-binding fragment thereof according to any one of [1] to [5], wherein the antibody or the antigen-binding fragment thereof has a neutralizing activity (EC50) of 0.05 µg/mL (approximately 0.33 nM) or lower against the *Clostridium difficile* toxin A in measurement using a human lung fibroblast IMR-90.
[8] An antibody capable of specifically binding to *Clostridium difficile* toxin B protein and neutralizing its biological activity, or an antigen-binding fragment thereof, containing:
   (i)
      (a) heavy chain CDR1 comprising the amino acid sequence of SEQ ID NO: 16 or an amino acid sequence deviated from the amino acid sequence of SEQ ID NO: 16 by deletion, substitution, insertion, and/or addition mutations of one to several amino acid residues,
      (b) heavy chain CDR2 comprising the amino acid sequence of SEQ ID NO: 17 or an amino acid sequence deviated from the amino acid sequence of SEQ ID NO: 17 by deletion, substitution, insertion, and/or addition mutations of one to several amino acid residues, and
      (c) heavy chain CDR3 comprising the amino acid sequence of SEQ ID NO: 18 or an amino acid sequence deviated from the amino acid sequence of SEQ ID NO: 18 by deletion, substitution, insertion, and/or addition mutations of one to several amino acid residues; and
   (ii)
      (a) light chain CDR1 comprising the amino acid sequence of SEQ ID NO: 22 or an amino acid sequence deviated from the amino acid sequence of SEQ ID NO: 22 by deletion, substitution, insertion, and/or addition mutations of one to several amino acid residues,
      (b) light chain CDR2 comprising the amino acid sequence of SEQ ID NO: 23 or an amino acid sequence deviated from the amino acid sequence of SEQ ID NO: 23 by deletion, substitution, insertion, and/or addition mutations of one to several amino acid residues, and
      (c) light chain CDR3 comprising the amino acid sequence of SEQ ID NO: 24 or an amino acid sequence deviated from the amino acid sequence of SEQ ID NO: 24 by deletion, substitution, insertion, and/or addition mutations of one to several amino acid residues.
[9] The antibody or an antigen-binding fragment thereof according to [8], comprising:
   (i)
      (a) the amino acid sequence of heavy chain CDR1 comprising the amino acid sequence of SEQ ID NO: 16,
      (b) the amino acid sequence of heavy chain CDR2 comprising the amino acid sequence of SEQ ID NO: 17, and
      (c) the amino acid sequence of heavy chain CDR3 comprising the amino acid sequence of SEQ ID NO: 18; and
   (ii)
      (a) the amino acid sequence of light chain CDR1 comprising the amino acid sequence of SEQ ID NO: 22,
      (b) the amino acid sequence of light chain CDR2 comprising the amino acid sequence of SEQ ID NO: 23, and
      (c) the amino acid sequence of light chain CDR3 comprising the amino acid sequence of SEQ ID NO: 24.
[10] The antibody or an antigen-binding fragment thereof according to [8] or [9], comprising
   (a) a heavy chain variable region (VH) comprising the amino acid sequence of SEQ ID NO: 15 or an amino acid sequence having 95% or higher identity to the amino acid sequence of SEQ ID NO: 15, and
   (b) a light chain variable region (VL) comprising the amino acid sequence of SEQ ID NO: 21 or an amino acid sequence having 95% or higher identity to the amino acid sequence of SEQ ID NO: 21.
[11] The antibody or an antigen-binding fragment thereof according to any one of [8] to [10], comprising
   (a) a heavy chain variable region (VH) comprising the amino acid sequence of SEQ ID NO: 15, and
   (b) a light chain variable region (VL) comprising the amino acid sequence of SEQ ID NO: 21.
[12] The antibody or an antigen-binding fragment thereof according to any one of [8] to [11], wherein the antibody or the antigen-binding fragment thereof is of IgG1 (λ) class (subclass).
[13] The antibody or an antigen-binding fragment thereof according to any one of [8] to [12], wherein the antibody or the antigen-binding fragment thereof has a neutralizing activity (EC50) of 0.1 µg/mL (approximately 0.7 nM) or lower against the *Clostridium difficile* toxin B in measurement using a human lung fibroblast IMR-90.
[14] A pharmaceutical composition comprising an antibody or an antigen-binding fragment thereof according to any one of [1] to [13] and a pharmaceutically acceptable carrier.
[15] The pharmaceutical composition according to [14], wherein the pharmaceutical composition comprises
   (a) a first antibody specifically binding to *Clostridium difficile* toxin A protein or an antigen-binding fragment thereof according to any one of [1] to [7], and
   (b) a second antibody specifically binding to *Clostridium difficile* toxin B protein or an antigen-binding fragment thereof according to any one of [8] to [13].
[16] The pharmaceutical composition according to [15], wherein the first and second monoclonal antibodies or antigen-binding fragments thereof neutralize the *Clostridium difficile* toxin A and the *Clostridium difficile* toxin B, respectively, *in vitro* or *in vivo.*
[17] The pharmaceutical composition according to any one of [14] to [16], wherein the pharmaceutical composition is intended for the treatment of *Clostridium difficile* infection.
[18] An isolated nucleic acid encoding the amino acid sequence of an antibody or an antigen-binding fragment thereof according to any one of [1] to [7], an isolated nucleic acid comprising a nucleotide sequence of SEQ ID NO: 1 or 7, or an isolated nucleic acid hybridizing under high stringent conditions to any of these nucleic acids.
[19] A vector comprising an isolated nucleic acid according to [18] therein.
[20] A host cell comprising a recombinant expression vector according to [19] introduced thereinto.
[21] An isolated nucleic acid encoding the amino acid sequence of an antibody or an antigen-binding fragment thereof according to any one of [8] to [13], an isolated nucleic acid comprising a nucleotide sequence of SEQ ID NO: 13 or 19, or an isolated nucleic acid hybridizing under high stringent conditions to any of these nucleic acids.
[22] A vector comprising an isolated nucleic acid according to [21] therein.
[23] A host cell comprising a recombinant expression vector according to [22] introduced thereinto.

### ADVANTAGEOUS EFFECTS OF INVENTION

The anti-toxin A antibody and the anti-toxin B antibody of the present invention or the antigen-binding fragments thereof specifically bind to toxin A and toxin B, respectively, and cancel (neutralize) their biological activity. Thus, the anti-toxin A antibody and the anti-toxin B antibody of the present invention or the antigen-binding fragments thereof can be expected to have therapeutic or preventive effects on CDI. Also, in an embodiment, the anti-toxin A antibody and the anti-toxin B antibody of the present invention are human monoclonal antibodies and thus probably have low immunogenicity.

### BRIEF DESCRIPTION OF DRAWINGS

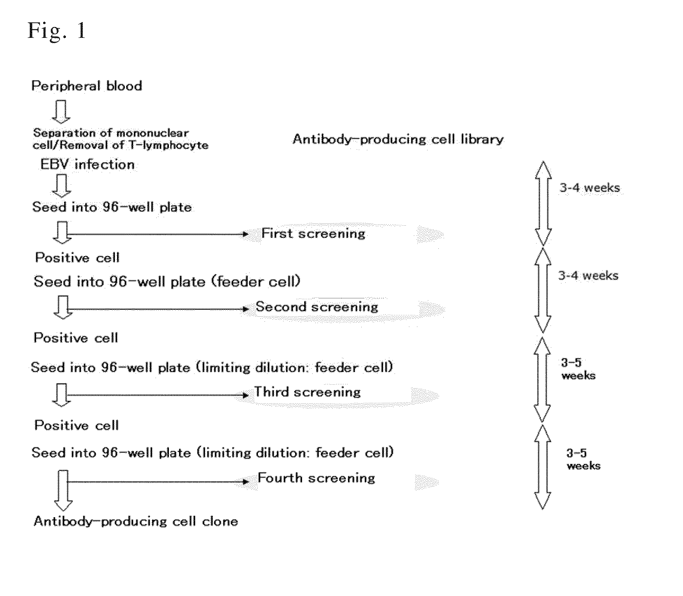

[Figure 1] Figure 1 is a diagram showing, in the form of flow chart, typical procedures of separating an antibody-producing cell clone producing the anti-C. *difficile* toxin antibody according to the present invention.

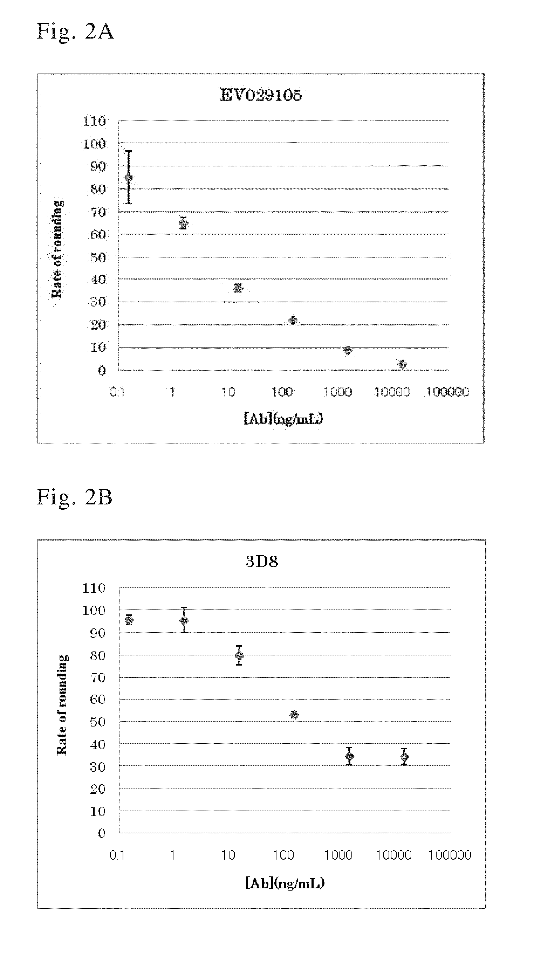

[Figure 2A] Figures 2A to 2D are a set of graphs showing results of *in vitro* neutralization assay carried out in IMR-90 cells in the presence of an anti-toxin A monoclonal antibody. The number of inoculated cells was set to 3.5 x 10⁴ per well, and the concentration of toxin A was set to 20 ng/mL. For each antibody, the number of rounded cells with the addition of the toxin and without the addition of the antibody was defined as 100, while the number of rounded cells without the addition of the toxin and without the addition of the antibody was defined as 0. The cells were counted in three or more images taken for one field of view, and an average was calculated. Figure 2A shows the neutralizing activity of EV029105a against toxin A. In the diagram, the ordinate indicates the rate of rounding in percentage, and the abscissa indicates antibody concentration (ng/mL).
[Figure 2B] Figures 2A to 2D are a set of graphs showing results of *in vitro* neutralization assay carried out in IMR-90 cells in the presence of an anti-toxin A monoclonal antibody. The number of inoculated cells was set to 3.5 × 10⁴ per well, and the concentration of toxin A was set to 20 ng/mL. For each antibody, the number of rounded cells with the addition of the toxin and without the addition of the antibody was defined as 100, while the number of rounded cells without the addition of the toxin and without the addition of the antibody was defined as 0. The cells were counted in three or more images taken for one field of view, and an average was calculated. Figure 2B shows the neutralizing activity of 3D8 against toxin A. In the diagram, the ordinate indicates the rate of rounding in percentage, and the abscissa indicates antibody concentration (ng/mL).

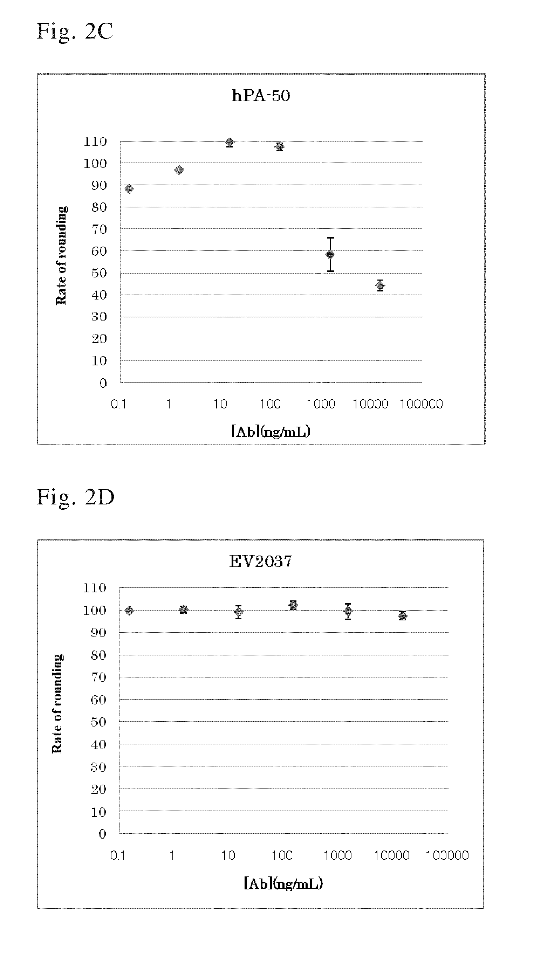

[Figure 2C] Figures 2A to 2D are a set of graphs showing results of *in vitro* neutralization assay carried out in IMR-90 cells in the presence of an anti-toxin A monoclonal antibody. The number of inoculated cells was set to 3.5 × 10⁴ per well, and the concentration of toxin A was set to 20 ng/mL. For each antibody, the number of rounded cells with the addition of the toxin and without the addition of the antibody was defined as 100, while the number of rounded cells without the addition of the toxin and without the addition of the antibody was defined as 0. The cells were counted in three or more images taken for one field of view, and an average was calculated. Figure 2C shows the neutralizing activity of hPA-50 against toxin A. In the diagram, the ordinate indicates the rate of rounding in percentage, and the abscissa indicates antibody concentration (ng/mL).
[Figure 2D] Figures 2A to 2D are a set of graphs showing results of *in vitro* neutralization assay carried out in IMR-90 cells in the presence of an anti-toxin A monoclonal antibody. The number of inoculated cells was set to 3.5 × 10⁴ per well, and the concentration of toxin A was set to 20 ng/mL. For each antibody, the number of rounded cells with the addition of the toxin and without the addition of the antibody was defined as 100, while the number of rounded cells without the addition of the toxin and without the addition of the antibody was defined as 0. The cells were counted in three or more images taken for one field of view, and an average was calculated. Figure 2D shows the neutralizing activity of EV2037 against toxin A. In the diagram, the ordinate indicates the rate of rounding in percentage, and the abscissa indicates antibody concentration (ng/mL).

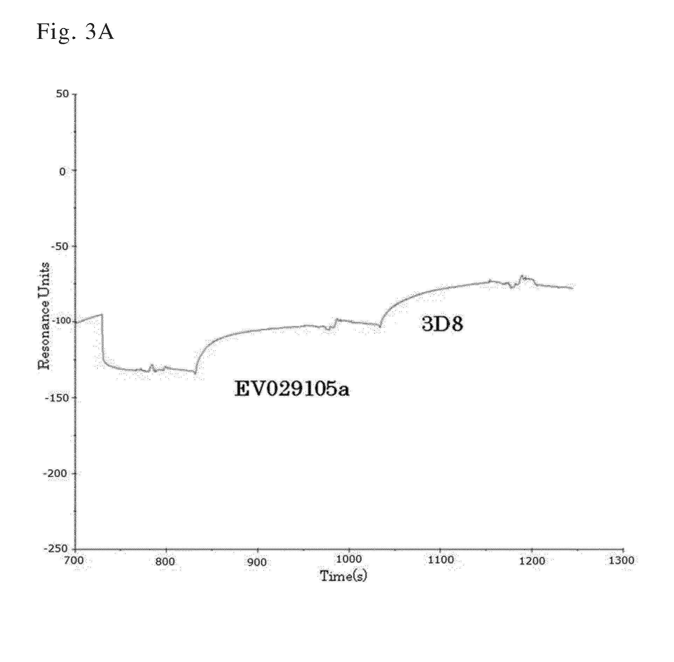

[Figure 3A] Figures 3A to 3C are a set of graphs showing the competitive binding of 3 anti-toxin A monoclonal antibodies (EV029105a, 3D8, and hPA-50) to toxin A using Biacore. To a sensor chip CAP, biotinylated toxin A was added, subsequently a first antibody was added, and finally a second antibody was added. In this competition assay, Biacore T200(R) apparatus was used. Figure 3A shows results about the combined use of "EV029105a and 3D8" as the first and second antibodies. In the diagram, the ordinate indicates resonance units, and the abscissa indicates time (sec).

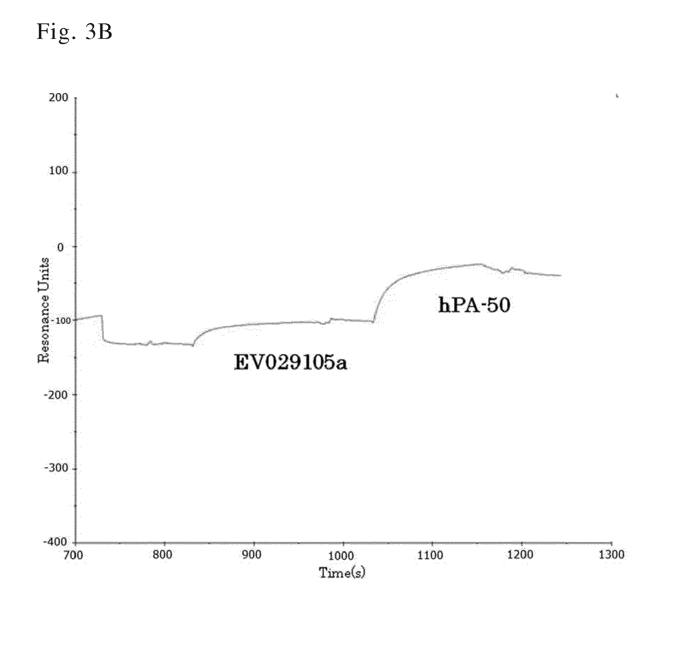

[Figure 3B] Figures 3A to 3C are a set of graphs showing the competitive binding of 3 anti-toxin A monoclonal antibodies (EV029105a, 3D8, and hPA-50) to toxin A using Biacore. To a sensor chip CAP, biotinylated toxin A was added, subsequently a first antibody was added, and finally a second antibody was added. In this competition assay, Biacore T200(R) apparatus was used. Figure 3B shows results about the combined use of "EV029105a and hPA-50" as the first and second antibodies. In the diagram, the ordinate indicates resonance units, and the abscissa indicates time (sec).

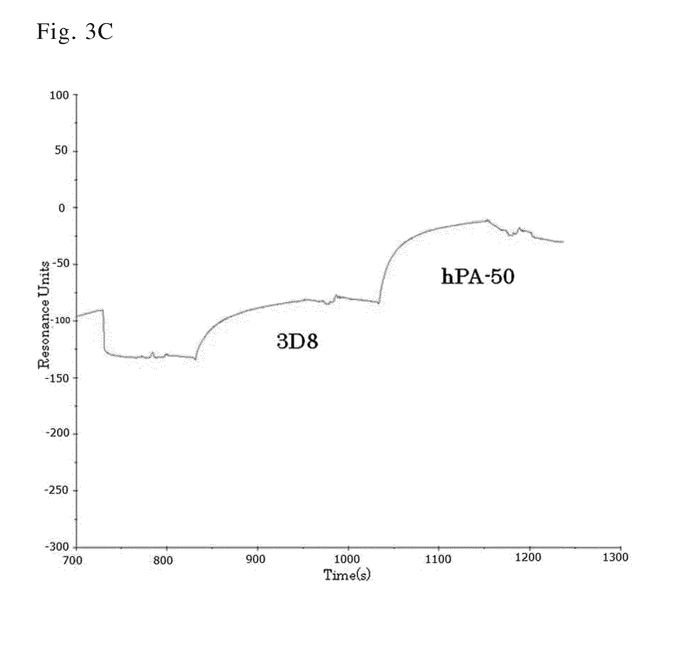

[Figure 3C] Figures 3A to 3C are a set of graphs showing the competitive binding of 3 anti-toxin A monoclonal antibodies (EV029105a, 3D8, and hPA-50) to toxin A using Biacore. To a sensor chip CAP, biotinylated toxin A was added, subsequently a first antibody was added, and finally a second antibody was added. In this competition assay, Biacore T200(R) apparatus was used. Figure 3C shows results about the combined use of "3D8 and hPA-50" as the first and second antibodies. In the diagram, the ordinate indicates resonance units, and the abscissa indicates time (sec).

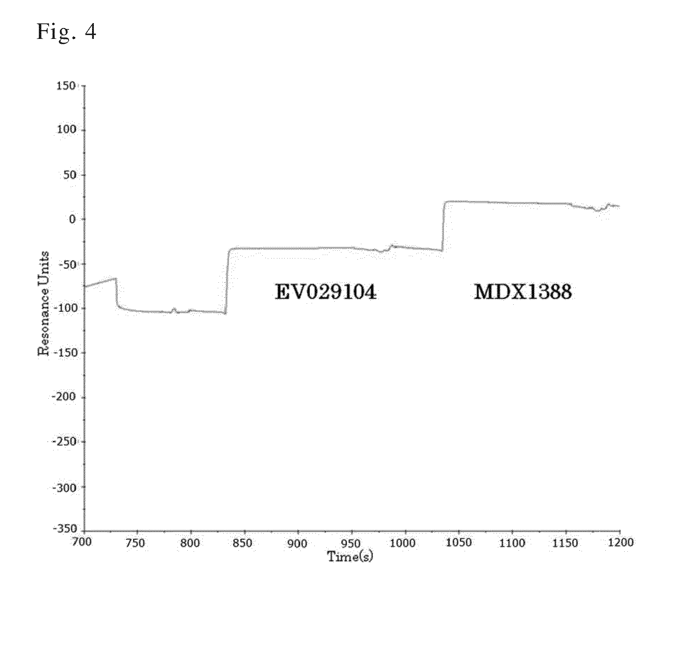

[Figure 4] Figure 4 is a graph showing the binding of anti-toxin B monoclonal antibodies (EV029104 and MDX1388) to toxin B in the epitope analysis of each antibody by competition assay using Biacore. To a sensor chip CAP, biotinylated toxin B was added, subsequently the EV029104 antibody was added, and finally the MDX1388 antibody was added. In this competition assay, Biacore T200(R) apparatus was used.

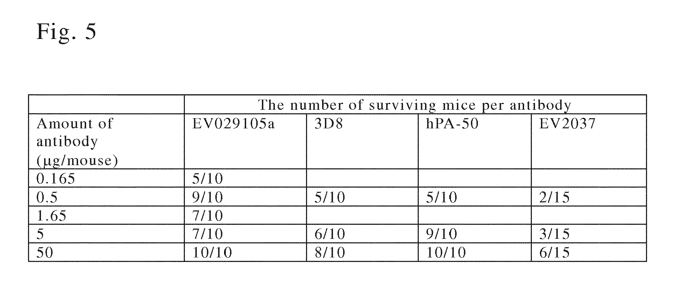

[Figure 5] Figure 5 shows the protective effect of an anti-toxin A antibody against mouse lethality by toxin A. The number of surviving mice is indicated in numerator, and the number of mice in an administration group is indicated in denominator.

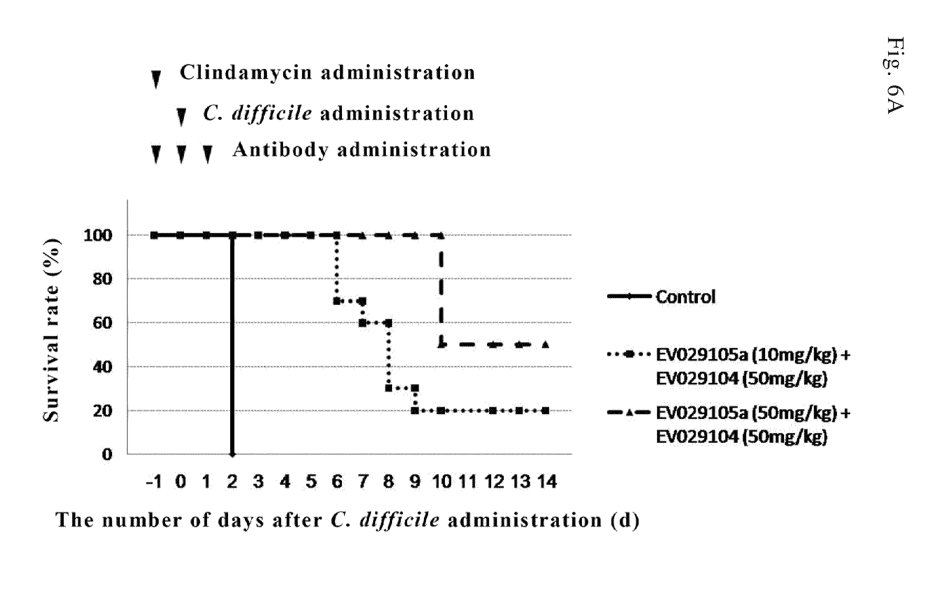

[Figure 6A] Figure 6A shows the protective effects of an anti-toxin A antibody and an anti-toxin B antibody against the lethality of hamsters by *C. difficile.* Figure 6A shows results of comparing antibody administration groups with a group given only clindamycin without the administration of the antibody, wherein the antibody administration groups were set to two groups: a group given EV029105a and EV029104 both at 50 mg/kg per dose; and a group given EV029105a at 10 mg/kg and EV029104 at 50 mg/kg. The ordinate indicates the survival rate of hamsters, and the abscissa indicates the number of days after *C. difficile* administration.

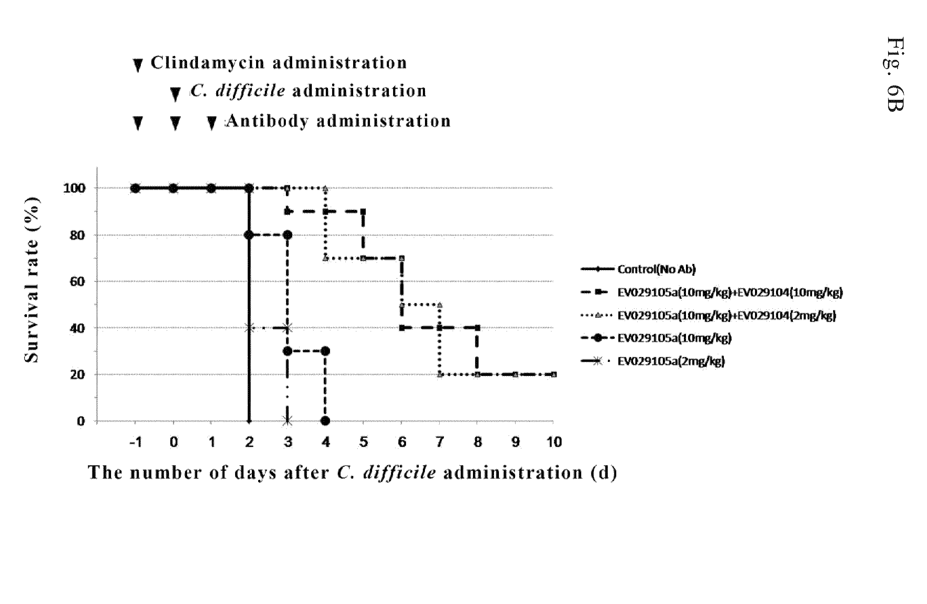

[Figure 6B] Figure 6B shows the protective effects of an anti-toxin A antibody and an anti-toxin B antibody against the lethality of hamsters by *C. difficile.* Figure 6B shows results of comparing antibody administration groups with a group given only clindamycin without the administration of the antibody, wherein the dose of the antibody was decreased and the antibody administration groups were set to four groups: a group given EV029105a at 10 mg/kg and EV029104 at 10 mg/kg per dose; a group given EV029105a at 10 mg/kg and EV029104 at 2 mg/kg; a group given only EV029105a at 10 mg/kg; and a group given only EV029105a at 2 mg/kg. The ordinate indicates the survival rate of hamsters, and the abscissa indicates the number of days after *C. difficile* administration.

### DESCRIPTION OF EMBODIMENTS

### 1. Definition

In the present specification, scientific terms and technical terms used in relation to the present invention have meanings generally understood by those skilled in the art. Words in the singular form shall be construed to include the plural and vice versa, unless the context otherwise requires. In general, nomenclatures used in relation to cells and techniques of tissue culture, molecular biology, immunology, microbiology, genetics, protein and nucleic acid chemistries, and hybridization described herein are well known in the art and generally used.

The terms used in the present invention are as defined below, unless otherwise specified.

### 1) Clostridium difficile (or C. difficile)

This bacterium is an obligately anaerobic gram-positive sporulating rod. The bacteria belonging to the genus *Clostridium* include, in addition to *C. difficile, Clostridium tetani* (tetanus bacillus), *Clostridium botulinum*, *Clostridium perfringens*, and the like. All of these bacteria are known to cause serious diseases. Until around the mid-1970s, the onset of pseudomembranous colitis (PMC) was recognized as being found particularly after use of some antibacterial drugs such as clindamycin and lincomycin. In recent years, however, this pseudomembranous colitis has been shown to be an inflammation in the large intestine that is manifested in response to toxins produced by *C. difficile* that has grown along with change in live bacterial flora in the intestine caused by the administration of antibiotics. *C. difficile* includes strains that produce no toxin, strains that produce low levels of toxins, and strains that produce high levels of toxins. Particularly, since 2003, a rise in the morbidity of C. *difficile* infection caused by the spread of hospital-acquired infection by a BI/NAP1/027 strain or the like having high toxin productivity, and the resulting rise in death rate have been major social problems.

### 2) C. difficile infection (CDI)

CDI is an infection by C. *difficile* and a series of following digestive system diseases caused by toxin production. The toxins produced by *C. difficile* act on the large intestinal epithelium to cause various degrees of damages from mild diarrhea to severe colitis. The most severe CDI cases are cases affected by pseudomembranous colitis (PMC) accompanied by terrible diarrhea, abdominal pain, and systemic signs such as fever, resulting in reportedly high fatality. Moderately severe cases are found to have terrible diarrhea, abdominal pain and tenderness, systemic signs (e.g., fever), and leukocytosis, etc., and also called antibiotic-associated colitis (AAC). Intestinal injury in AAC is less severe than that in PMC. AAC is free from endoscopic appearance characteristic of the colon in PMC and results in low death rate. Mild CDI cases are characterized by mild to moderate diarrhea associated with antibiotic administration. These mild cases are found to be free from an inflammation in the large intestine and systemic signs such as fever and also called antibiotic-associated diarrhea (AAD).

### 3) C. difficile toxin

The toxins produced by *C. difficile* are mainly toxin A and toxin B proteins. The "toxin A" (or also referred to as *"C. difficile* toxin A") is a protein encoded by a gene called tcdA on the *C. difficile* gene. Its amino acid sequence (SEQ ID NO: 25) has been registered under Accession No. P16154 with GenBank. The "toxin B" (or also referred to as *"C. difficile* toxin B") is a protein encoded by a gene called tcdB on the *C. difficile* gene. Its amino acid sequence (SEQ ID NO: 26) has been registered under Accession No. Q46034 with GenBank. Both of these genes are located in a 19.6-kb gene region called Paloc (pathogenicity locus). In addition to tcdA and tcdB, genes, such as tcdC (negative regulator) and tcdD (positive regulator), which are involved in the control of the expression thereof, are present on this gene region.

The "toxin A" is a 308-kDa enterotoxin produced extracellularly by *C. difficile.* This protein is composed of 2710 amino acid residues and broadly constituted by 4 domains. Particularly, a repeat structure of amino acid sequences (CRD: C-terminal repetitive domain) is present in approximately 1/3 of the C-terminal side of the toxin A. This domain is considered to play a role in recognizing and binding to glycoproteins on cell surface and is also called receptor-binding domain (or RB domain). On the other hand, the amino-terminal region has glucosyltransferase activity targeting the Rho/Ras superfamily having GTPase activity and contains an enzyme domain (glucosyltransferase domain; also referred to as a GT domain) that glycosylates these targeted enzymes and blocks their phosphorylating ability. The activity is considered to destroy the integrity of actin polymerization and cytoskeleton of cells (Eichel-Streiber, Trends Microbiol., 1996 (4) p. 375-382). The N-terminal side of the intermediate moiety of the toxin protein contains a cysteine protease domain (also referred to as a CP domain) and is considered to participate in processing to separate the GT domain from the toxin protein. Also, the C-terminal side of the intermediate moiety contains a highly hydrophobic region probably involved in the passage of the toxin protein through the membrane and is called membrane insertion domain (Jank, Trends Microbiol., 2008 (16) p. 222-229; and Hussack, Toxins 2010 (2) p. 998-1018). For information, anti-toxin A antibodies 3D8 and hPA-50 have both been reported to bind to the RB domain present on the C-terminal side of the toxin A.

Likewise, the "toxin B" is a 269-kDa enterotoxin produced extracellularly by *C. difficile.* This protein is composed of 2366 amino acid residues, and its amino acid sequence has approximately 60% homology to the amino acid sequence of the "toxin A". The protein is broadly constituted by 4 domains, as in the "toxin A", and has an RB domain and a GT domain similar to those in the toxin A. An anti-toxin B antibody MDX1388 has been reported to bind to the RB domain present on the C-terminal side of the toxin B, while another anti-toxin B antibody hPA-41 has been reported to bind to the N-terminal side of the toxin B. Both of these toxins cause *in vitro* a cytopathic effect (CPE) on cultured cells such as Vero cells, human lung fibroblasts IMR-90, human colon epithelial cells T-84, and CHO-K1 cells, and the looping of intestinal tracts. It is also known that the toxins lead to death by *in vivo* exposure thereto or depending on the doses of the toxins in evaluation systems using models such as hamsters.

### 4) Anti-C. difficile toxin antibody

This antibody binds to a toxin protein (e.g., toxin A or toxin B) produced by *C. difficile* and is capable of binding to an epitope site, for example, a linear epitope or a conformational epitope, in the toxin protein, or a fragment of the toxin protein, etc.

In the present specification, the "anti-*C*. *difficile* toxin antibody", the "antibody capable of neutralizing a *C**.** difficile* toxin", or the "antibody capable of neutralizing the biological activity of a *C. difficile* toxin" refers to an antibody that inhibits the biological activity of the *C. difficile* toxin through binding to the *C. difficile* toxin.

The term "disease caused by a *C. difficile* toxin" used herein includes a disease for which the *C. difficile* toxin in a test subject affected by the disease has been shown or considered to be responsible for the pathophysiology of the disease or to contribute to deterioration in the disease, and also includes other diseases. Thus, the disease caused by a *C. difficile* toxin is a disease whose symptoms and/or progression are presumably alleviated by the inhibition of the biological activity of the *C. difficile* toxin. This disease corresponds to the aforementioned *C. difficile* infection (CDI).

The symptoms of CDI can be alleviated or cured, for example, by use of the aforementioned anti-*C*. *difficile* toxin antibody. Specifically, the symptoms of the disease can be alleviated or cured by raising the anti-*C*. *difficile* toxin antibody concentration in the body fluid of a test subject affected by the disease (e.g., raising the anti-*C*. *difficile* toxin antibody concentration in the serum, plasma, or synovial fluid of the test subject).

### 5) Antibody

The term "antibody" used herein refers to an immunoglobulin molecule composed of four polypeptide chains, i.e., two heavy (H) chains and two light (L) chains, linked via disulfide bonds. The monoclonal antibody according to the present invention is also composed of an immunoglobulin molecule comprising two heavy chains (H chains) and two light chains (L chains). Each H chain is composed of an H chain variable region (also referred to as "HCVR" or "VH") and an H chain constant region (the H chain constant region is composed of three domains, which are also referred to as "CH1", "CH2", and "CH3", respectively (collectively referred to as CH)). Each L chain is composed of an L chain variable region (also referred to as "LCVR" or "VL") and an L chain constant region (the L chain constant region is composed of one domain, which is also referred to as "CL"). A region located before each constant region (also referred to as constant part, invariable region, or invariable part region) is called variable region (also referred to as variable part or variable part region).

Particularly, VH and VL are important because of their involvement in the binding specificity of the antibody for the antigen. Since the antibody interacts with its target antigen mainly through the amino acid residues of VH and VL, the amino acid sequences within the variable part regions differ more largely among individual antibodies than sequences without the variable part regions. VH and VL can be further subdivided into regions called framework regions (FRs) conserved among various antibodies, and hypervariable regions called complementarity-determining regions (CDRs). Each of VH and VL has three CDRs and four FRs, and these regions are arranged from the amino terminus to the carboxy terminus in the order of FR1, CDR1, FR2, CDR2, FR3, CDR3, and FR4.

### 6) "Antigen-binding fragment" of the antibody (or simply referred to as "antibody fragment")

The term "antigen-binding fragment" of the antibody (or simply referred to as an "antibody fragment") used herein refers to one or more antibody fragments (e.g., VH) having the ability to specifically bind to the antigen (*C*. *difficile* toxin). The fragment also includes a peptide having the minimum amino acid sequence specifically binding to the antigen. Examples of binding moieties included in the term "antigen-binding fragment" of the antibody include (i) a Fab fragment, (ii) a F(ab')2 fragment, (iii) an Fd fragment composed of VH and CH1 domains, (iv) an Fv fragment composed of VL and VH domains on a single arm of the antibody, (v) a dAb fragment composed of a VH domain (Ward et al., Nature 341: 544-546, 1989), (vi) isolated complementarity-determining regions (CDRs) having frameworks sufficient for the specific binding, and (vii) a bispecific antibody and (viii) a multispecific antibody composed of any combination of these fragments (i) to (vi). In the present specification, the term "antibody" used without discrimination includes not only whole antibodies but also these "antigen-binding fragments".

### 7) Isotype

The isotype refers to the class (e.g., IgM or IgG1) of an antibody encoded by a heavy chain constant region gene. The antibody according to the present invention is preferably of IgG1 (κ) or IgG1 (λ) class (subclass).

### 8) Specific binding

The term "specifically binding" or "specific binding" used herein refers to the recognition of the predetermined antigen so as to bind thereto.

Typically, the dissociation constant (K_{D} value) of the anti-C. *difficile* toxin A antibody of the present invention against the toxin A is preferably 1 × 10⁻⁷ M or lower, more preferably 1 × 10⁻⁸ M or lower, further preferably 1 × 10⁻⁹ M or lower, most preferably 2 × 10⁻¹⁰ M or lower. The dissociation constant of the antibody against the toxin A can be measured using a method known in the art. For example, the dissociation constant can be measured against toxin A immobilized on a chip by use of a protein interaction analysis apparatus such as Biacore T200(R).

### 9) Biological activity of antibody

The biological properties of the antibody or an antibody composition can be evaluated by testing the ability of the antibody to suppress *in vitro* the biological activity of the *C*. *difficile* toxin according to an assay method well known to those skilled in the art. The *in vitro* assay method includes a binding assay method such as ELISA, and a neutralization assay method, etc. In an *in vivo* evaluation system, its effectiveness for humans can be predicted from the ability of the anti-toxin antibody to protect an animal model against lethal attack by *C. difficile.* Specific examples of the animal model for predicting the effectiveness are described herein and further include an intestinal ligation model (WO2006/121422) and the like.

The terms such as "neutralization", "inhibitory effect", "inhibition", "suppression", "capable of inhibiting", and "protection" used herein mean that biological activity attributed to the antigen (*C*. *difficile* toxin) is reduced by approximately 5 to 100%, preferably 10 to 100%, more preferably 20 to 100%, more preferably 30 to 100%, more preferably 40 to 100%, more preferably 50 to 100%, more preferably 60 to 100%, more preferably 70 to 100%, further preferably 80 to 100%.

The neutralizing ability of the anti-*C*. *difficile* toxin antibody can be evaluated using, for example, human lung fibroblasts IMR-90, by culturing, for 2 days, the cells exposed to toxin A and examining the resulting rate of rounding (cytopathic effect: CPE) of the cells in the presence of the anti-toxin A antibody.

The anti-C. *difficile* toxin A antibody of the present invention or the antigen-binding fragment thereof preferably has the ability to block approximately 50% cell rounding (EC50) at 1 µg/mL (approximately 7 nM) or lower, more preferably 0.1 µg/mL (approximately 0.7 nM) or lower, further preferably 0.05 µg/mL (approximately 0.33 nM) or lower, still further preferably 0.01 µg/mL (approximately 0.07 nM) or lower, most preferably approximately 0.005 µg/mL (approximately 0.033 nM) or lower, as the neutralizing activity against the toxin A using toxin A-exposed IMR-90 cells.

The anti-*C*. *difficile* toxin B antibody of the present invention or the antigen-binding fragment thereof preferably has the ability to block approximately 50% cell rounding (EC50) at 1 µg/mL (approximately 7 nM) or lower, more preferably 0.1 µg/mL (approximately 0.7 nM) or lower, as the neutralizing activity against the toxin B using toxin B-exposed IMR-90 cells.

### 10) Animal model experiment and therapeutically effective amount

The ability of the antibody against measurable clinical parameters can be evaluated in an animal model system for predicting its effectiveness for humans. For example, the effectiveness for humans can be predicted from the ability of the anti-toxin antibody to protect a mouse against lethal attack by *C. difficile.* The amount of the anti-toxin antibody effective for the treatment of CDI or the "therapeutically effective amount" refers to the amount of the antibody effective for suppressing CDI in a subject when the antibody is administered at a single dose or plural doses to the subject. The therapeutically effective amount of the antibody or the antibody fragment may differ depending on factors such as the disease status, age, sex, and body weight of an individual, and the ability of the antibody or the antigen-binding fragment to induce the desired response in the individual. The therapeutically effective amount is an amount at which the therapeutically beneficial effect of the antibody or a moiety of the antibody exceeds its toxic effect or adverse effect.

The amount of the anti-toxin antibody effective for the prevention of the disease or the "preventively effective amount" of the antibody refers to the amount effective for hindering or delaying the onset or recurrence of CDI or for suppressing its symptoms when the antibody is administered at a single dose or plural doses to the subject. If longer prevention is desired, the antibody can be administered at an increased dose.

Example 12 of the present application demonstrated the *in vivo* protective effect of the anti-toxin A antibody against the lethal action of the toxin A on a mouse. In Example 12, EV029105a of the present invention intraperitoneally administered at 0.165 µg/head (approximately 8 µg/kg) was shown to have the activity of neutralizing 50% lethal action of toxin A administered at 200 ng/head. Also, EV029105a administered at 0.5 µg/head (approximately 25 µg/kg) was shown to be evidently superior in preventive effect to 3D8 or hPA-50 administered at the same dose thereas. Specifically, the anti-toxin A antibody according to the present invention or the antigen-binding fragment thereof includes an antibody having the activity of neutralizing 50% or more lethal action of toxin A administered at 200 ng/head when intraperitoneally administered at 0.165 µg/head (approximately 8 µg/kg or approximately 55 pmol/kg), and an antibody evidently superior in preventive effect to 3D8 or hPA-50 administered at the same dose thereas when administered at 0.5 µg/head (approximately 25 µg/kg or approximately 170 pmol/kg).

Furthermore, Example 13 demonstrated the remarkable protective effects of the anti-toxin A antibody and the anti-toxin B antibody against the lethal action of C. *difficile* on a Syrian golden hamster. Particularly, as shown in Figure 6B, the combined administration of EV029105a and EV029104 was shown to have a very remarkable and dose-dependent protective effect against CDI, even as compared with the administration of EV029105a alone. Specifically, in one aspect, the present invention relates to a combined administration method using the anti-toxin A antibody or the antigen-binding fragment thereof and the anti-toxin B antibody or the antigen-binding fragment thereof. In this method, the dose of the first monoclonal antibody or the antigen-binding fragment thereof against the toxin A is preferably 1 mg/kg (approximately 6.7 nmol/kg) or larger, more preferably 10 mg/kg (approximately 67 nmol/kg) or larger, for example, when EV029105a is administered at a single dose or plural doses. In addition, the dose of the second monoclonal antibody or the antigen-binding fragment thereof used in combination therewith against the toxin B is preferably 1 mg/kg (approximately 6.7 nmol/kg) or larger, more preferably 10 mg/kg (approximately 67 nmol/kg) or larger, for example, when EV029104 is administered at a single dose or plural doses. However, the ranges of such doses are given merely for illustrative purposes and are not intended to limit the scope of the present invention. Depending on the type of a composition, one dose may be continuously administered using an infusion pump or the like, or divided doses may be administered at a given interval. It is well known that various protocols are possible as to doses, dosing schedules, modes of administration, and administration sites depending on the pathological conditions of a test subject, etc. The first monoclonal antibody or the antigen-binding fragment thereof is not limited to one type selected from the antibody group, and plural types may be selected. Likewise, one type of second monoclonal antibody or antigen-binding fragment thereof may be selected from the antibody group, or plural types may be selected depending on results of combination.

### 11) Procedure for C. difficile infection (CDI)

The procedure for CDI means that the anti-toxin antibodies of the present invention or the antigen-binding fragments thereof are administered each alone or in combination, or together with a drug (e.g., an antibody drug or an antibiotic) other than the present invention, in order to treat or prevent CDI. The subject may be a patient infected by *C. difficile* or having the symptoms (e.g., diarrhea, colitis, and abdominal pain) of CDI or a predisposition of CDI (e.g., being treated with antibiotics, or having a history of CDI and a risk of recurrence of the disease). The procedure can be to treat, cure, alleviate, reduce, change, repair, ameliorate, lessen, or improve the symptoms of a disease associated with the infection or a predisposition of the disease, or to influence any of these procedures.

### 12) substantially identical

In relation to the term "substantially identical" used herein, the deletion, substitution, insertion, or addition of one or more amino acid residues in the amino acid sequence of the antibody of the present invention, or the combination of any two or more of these means that one or more amino acid residues are deleted, substituted, inserted, or added at one or more arbitrary positions in the same amino acid sequence, and two or more of the deletion, the substitution, the insertion, and the addition may occur at the same time.

Amino acids constituting proteins in the natural world can be grouped according to the properties of their side chains and can be classified into amino acid groups having similar properties, for example, groups of aromatic amino acids (tyrosine, phenylalanine, and tryptophan), basic amino acids (lysine, arginine, and histidine), acidic amino acids (aspartic acid and glutamic acid), neutral amino acids (serine, threonine, asparagine, and glutamine), hydrocarbon chain-containing amino acids (alanine, valine, leucine, isoleucine, and proline), and others (glycine, methionine, and cysteine).

As an example, amino acid residues that can be mutually substituted, also including nonnatural amino acids, may be grouped as follows, and the amino acid residues included in the same group can be mutually substituted: group A: leucine, isoleucine, norleucine, valine, norvaline, alanine, 2-aminobutanoic acid, methionine, o-methylserine, t-butylglycine, t-butylalanine, and cyclohexylalanine; group B: aspartic acid, glutamic acid, isoaspartic acid, isoglutamic acid, 2-aminoadipic acid, and 2-aminosuberic acid; group C: asparagine and glutamine; group D: lysine, arginine, ornithine, 2,4-diaminobutanoic acid, and 2,3-diaminopropionic acid; group E: proline, 3-hydroxyproline, and 4-hydroxyproline; group F: serine, threonine, and homoserine; and group G: phenylalanine, tyrosine, and tryptophan.

The identity of an amino acid sequence or a nucleotide sequence can be determined using the Karlin-Altschul algorithm BLAST (Proc. Natl. Acad. Sci. USA 872264-2268, 1990; and Proc Natl Acad Sci USA 90: 5873, 1993). A program called BLASTN or BLASTX based on the BLAST algorithm has been developed (Altschul SF, et al., J Mol Biol 215: 403, 1990). In the case of analyzing a nucleotide sequence using BLASTN, parameters are set to, for example, score = 100 and wordlength = 12. In the case of analyzing an amino acid sequence using BLASTX, parameters are set to, for example, score = 50 and wordlength = 3. In the case of using the BLAST and Gapped BLAST programs, default parameters of each program are used.

### 2. Antibody of the present invention or antigen-binding fragment thereof 1) Anti-toxin A antibody of the present invention or antigen-binding fragment thereof

In one aspect, the present invention provides an antibody capable of specifically binding to toxin A protein produced by C. *difficile* and neutralizing its biological activity, or an antigen-binding fragment thereof (hereinafter, referred to as the antibody of the present invention). In a more specific embodiment, the antibody of the present invention is an antibody capable of recognizing and binding to an epitope different from that for the anti-toxin A antibodies 3D8 and hPA-50, in the RB domain of toxin A and neutralizing the biological activity of the protein, or an antigen-binding fragment thereof.

In one embodiment, examples of the anti-toxin A antibody of the present invention include an antibody capable of specifically binding to toxin A protein produced by *C. difficile* and neutralizing its biological activity, wherein the antibody has the amino acid sequences of SEQ ID NOs: 4, 5, and 6 as the amino acid sequences of heavy chains CDR1, CDR2, and CDR3, respectively, and has the amino acid sequences of SEQ ID NOs: 10, 11, and 12 as the amino acid sequences of light chains CDR1, CDR2, and CDR3, respectively.

In another embodiment, examples of the anti-toxin A antibody of the present invention include an antibody substantially identical to the antibody of the preceding embodiment. Such an antibody includes an antibody capable of specifically binding to toxin A protein produced by *C. difficile* and neutralizing its biological activity, wherein the antibody has heavy chains CDR1 to CDR3 and light chains CDR1 to CDR3 composed of amino acid sequences substantially identical to SEQ ID NOs: 4, 5, and 6 and SEQ ID NOs: 10, 11, and 12, as the amino acid sequences of heavy chains CDR1, CDR2, and CDR3 and light chains CDR1, CDR2, and CDR3. Specifically, such an antibody has the neutralizing activity and can have, as heavy chain and light chain CDR sequences, amino acid sequences deviated from the amino acid sequences of SEQ ID NOs: 4, 5, and 6 and SEQ ID NOs: 10, 11, and 12 by deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues in each sequence, or the combination of two or more of these mutations. Its amino acid sequence except for the CDRs is not particularly limited, and a so-called CDR-grafted antibody having an amino acid sequence, except for CDRs, derived from another antibody, particularly, an antibody of different species is also encompassed by the antibody of the present invention. Of these antibodies, an antibody is preferred in which the amino acid sequence other than CDRs is also derived from a human. If necessary, its framework region (FR) may have the deletion, substitution, insertion, or addition of one or several (specific numbers are the same as above) amino acid residues, or the combination of two or more of these mutations.

In a further alternative embodiment, examples of the anti-toxin A antibody of the present invention include an antibody capable of specifically binding to toxin A protein produced by *C. difficile* and neutralizing its biological activity, comprising (a) a heavy chain variable region (VH) represented by the amino acid sequence of SEQ ID NO: 3; an amino acid sequence deviated from the amino acid sequence of SEQ ID NO: 3 by deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 95% or higher (preferably 96% or higher, 97% or higher, 98% or higher, 99% or higher, or 99.5% or higher) identity to the amino acid sequence of SEQ ID NO: 3, and (b) a light chain variable region (VL) represented by the amino acid sequence of SEQ ID NO: 9; an amino acid sequence deviated from the amino acid sequence of SEQ ID NO: 9 by deletion, substitution, insertion, or addition of one to several (specific numbers are the same as above) amino acid residues, or the combination of two or more of these mutations; or an amino acid sequence having 95% or higher (specific % is the same as above) identity to the amino acid sequence of SEQ ID NO: 9.

### 2) Anti-toxin B antibody of the present invention or antigen-binding fragment thereof

In one aspect, the present invention also provides an antibody capable of specifically binding to toxin B protein produced by *Clostridium difficile* and neutralizing its biological activity, or an antigen-binding fragment thereof. In a more specific embodiment, the present invention provides an antibody capable of specifically binding to an epitope different from that for the anti-toxin B antibodies MDX1388 and hPA-41, in the RB domain on the C-terminal side and neutralizing the biological activity of the protein, or an antigen-binding fragment thereof.

In one embodiment, examples of the anti-toxin B antibody of the present invention include an antibody capable of specifically binding to toxin B protein produced by *C. difficile* and neutralizing its biological activity, wherein the antibody has the amino acid sequences of SEQ ID NOs: 16, 17, and 18 as the amino acid sequences of heavy chains CDR1, CDR2, and CDR3, respectively, and has the amino acid sequences of SEQ ID NOs: 22, 23, and 24 as the amino acid sequences of light chains CDR1, CDR2, and CDR3, respectively.

In another embodiment, examples of the anti-toxin B antibody of the present invention include an antibody substantially identical to the antibody of the preceding embodiment. Such an antibody includes an antibody capable of specifically binding to toxin B protein produced by C. *difficile* and neutralizing its biological activity, wherein the antibody has heavy chains CDR1 to CDR3 and light chain CDR1 to CDR3 composed of amino acid sequences substantially identical to SEQ ID NOs: 16, 17, and 18 and SEQ ID NOs: 22, 23, and 24, as the amino acid sequences of heavy chains CDR1, CDR2, and CDR3 and light chains CDR1, CDR2, and CDR3. Specifically, such an antibody has the neutralizing activity and can have, as heavy chain and light chain CDR sequences, amino acid sequences deviated from the amino acid sequences of SEQ ID NOs: 16, 17, and 18 and SEQ ID NOs: 22, 23, and 24 by deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues in each sequence, or the combination of two or more of these mutations. The amino acid sequence except for the CDRs of the anti-toxin B antibody is not particularly limited, and a so-called CDR-grafted antibody having an amino acid sequence, except for CDRs, derived from another antibody, particularly, an antibody of different species is also encompassed by the antibody of the present invention. Of these antibodies, an antibody is preferred in which the amino acid sequence other than CDRs is also derived from a human. If necessary, its framework region (FR) may have the deletion, substitution, insertion, or addition of one or several (specific numbers are the same as above) amino acid residues, or the combination of two or more of these mutations.

In a further alternative embodiment, examples of the anti-toxin B antibody of the present invention include an antibody capable of specifically binding to toxin B protein produced by *C. difficile* and neutralizing its biological activity, comprising (a) a heavy chain variable region (VH) represented by the amino acid sequence of SEQ ID NO: 15; an amino acid sequence deviated from the amino acid sequence of SEQ ID NO: 15 by deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 95% or higher (preferably 96% or higher, 97% or higher, 98% or higher, 99% or higher, or 99.5% or higher) identity to the amino acid sequence of SEQ ID NO: 15, and (b) a light chain variable region (VL) represented by the amino acid sequence of SEQ ID NO: 21; an amino acid sequence deviated from the amino acid sequence of SEQ ID NO: 21 by deletion, substitution, insertion, or addition of one to several (specific numbers are the same as above) amino acid residues, or the combination of two or more of these mutations; or an amino acid sequence having 95% or higher (specific % is the same as above) identity to the amino acid sequence of SEQ ID NO: 21.

A method known in the art can be used as a method for preparing these antibodies (Riechmann L, et al., Reshaping human antibodies for therapy. Nature, 332:323-327, 1988). In the present invention, a completely human antibody is preferred, as a matter of course.

### 3. Nucleic acid encoding antibody of the present invention, etc.

In another aspect, the present invention provides a nucleic acid (nucleotide) encoding the anti-toxin A monoclonal antibody capable of specifically binding to toxin A and neutralizing its biological activity, or the antigen-binding fragment thereof. Examples of such a nucleic acid include a nucleic acid encoding an amino acid sequence selected from the group consisting of SEQ ID NOs: 2 to 6 and 8 to 12, and an isolated nucleic acid having high identity to the nucleic acid. In this context, the term "having high identity" means sequence identity to an extent that permits hybridization under high stringent conditions to the predetermined nucleic acid sequence and means having, for example, 60%, 70%, 80%, 90%, or 95% or higher identity. The present invention provides an isolated nucleic acid selected from nucleic acids hybridizing thereto under high stringent conditions. Preferably, the nucleic acid is DNA or RNA, more preferably DNA.

The present invention includes a nucleic acid (nucleotide) encoding the anti-toxin B monoclonal antibody capable of specifically binding to C. *difficile* toxin B and neutralizing its biological activity, or the antigen-binding fragment thereof and also includes a nucleic acid encoding an amino acid sequence selected from the group consisting of SEQ ID NOs: 15 to 18 and 20 to 24, and an isolated nucleic acid having high identity to the nucleic acid. In this context, the term "having high identity" means sequence identity to an extent that permits hybridization under high stringent conditions to the predetermined nucleic acid sequence and means having, for example, 60%, 70%, 80%, 90%, or 95% or higher identity. The present invention provides an isolated nucleic acid selected from nucleic acids hybridizing thereto under high stringent conditions. Preferably, the nucleic acid is DNA or RNA, more preferably DNA.

The "high stringent conditions" are conditions involving, for example, 5 × SSC, 5 × Denhardt's solution, 0.5% SDS, 50% formamide, and 50°C (see e.g., J. Sambrook et al., Molecular Cloning, A Laboratory Manual 2nd ed., Cold Spring Harbor Laboratory Press (1989), particularly, 11.45 "Conditions for Hybridization of Oligonucleotide Probes"). Under these conditions, it can be expected that a polynucleotide (e.g., DNA) having high identity is more efficiently obtained at a higher temperature. However, possible factors that influence the stringency of hybridization are a plurality of factors such as temperatures, probe concentrations, probe lengths, ionic strengths, times, and salt concentrations. Those skilled in the art can achieve similar stringency by appropriately selecting these factors.

The nucleic acid hybridizing under high stringent conditions includes a nucleic acid having, for example, 70% or higher, 80% or higher, 90% or higher, 95% or higher, 97% or higher, or 99% or higher identity to the nucleic acid encoding the amino acid sequence. The identity of a nucleotide sequence can be determined by use of the aforementioned identity search algorithm or the like (Proc. Natl. Acad. Sci. USA 872264-2268, 1990; and Proc Natl Acad Sci USA 90: 5873, 1993).

The nucleic acid preferred for the anti-toxin A antibody in the present invention is a nucleic acid having nucleotide sequences respectively encoding the amino acid sequences of SEQ ID NOs: 3 and 9, more preferably a nucleic acid having nucleotide sequences respectively encoding the amino acid sequences of SEQ ID NOs: 2 and 8 except for signal sequence moieties. The signal sequence moieties in SEQ ID NOs: 2 and 8 refer to amino acid sequences described to the left side of the moieties corresponding to the N-terminal amino acids in their respective variable region sequences (SEQ ID NOs: 3 and 9).

The nucleic acid is further preferably a nucleic acid comprising both of the nucleotide sequences of SEQ ID NOs: 1 and 7.

The nucleic acid preferred for the anti-toxin B antibody in the present invention is a nucleic acid having nucleotide sequences respectively encoding the amino acid sequences of SEQ ID NOs: 15 and 21, more preferably a nucleic acid having nucleotide sequences respectively encoding the amino acid sequences of SEQ ID NOs: 14 and 20 except for signal sequence moieties. The signal sequence moieties in SEQ ID NOs: 14 and 20 refer to amino acid sequences described to the left side of the moieties corresponding to the N-terminal amino acids in their respective variable region sequences (SEQ ID NOs: 15 and 21).

The nucleic acid is further preferably a nucleic acid comprising both of the nucleotide sequences of SEQ ID NOs: 13 and 19.

### 4. Vector, host cell, and antibody preparation method of the present invention

The present invention also relates to a vector comprising the nucleic acid incorporated therein and a host cell comprising the vector introduced thereinto, and a method for preparing the antibody using the vector and the host cell.

The antibody of the present invention can also be prepared as a recombinant human antibody by use of a method known in the art (see e.g., Nature, 312: 643,1984; and Nature, 321: 522, 1986). The antibody of the present invention can be prepared, for example, by culturing the host cell having the vector of the present invention incorporated therein and purifying the produced antibody from the culture supernatant or the like. More specifically, VH- and VL-encoding cDNAs can be respectively inserted to expression vectors for animal cells containing human antibody CH- and/or human antibody CL-encoding genes prepared from the same cell or another human cell to construct human antibody expression vectors, which are transferred to animal cells so that the antibody is produced by expression.

The vector to which the nucleic acid encoding VH or VL of the antibody of the present invention is incorporated is not necessarily limited and is preferably a vector that is routinely used for the expression of protein genes or the like and is particularly compatible with the expression of antibody genes, or a vector for high expression. Preferred examples thereof include vectors containing EF promoter and/or CMV enhancer. Usually, expression vectors respectively having VH- or VL-encoding nucleic acids incorporated therein are prepared, and host cells are cotransfected with the expression vectors. Alternatively, these nucleic acids may be incorporated in a single expression vector.

The host cell to which the expression vector is transferred is not necessarily limited and is preferably a cell that is routinely used for the expression of protein genes or the like and is particularly compatible with the expression of antibody genes. Examples thereof include bacteria (*E. coli,* etc.), *Actinomycetes,* yeasts, insect cells (SF9, etc.), and mammalian cells (COS-1, CHO, myeloma cells, etc.).

For the industrial production of the antibody, a recombinant animal cell line, for example, a CHO cell line, stably highly producing the antibody is generally used. The preparation and cloning of such a recombinant cell line, gene amplification for high expression, and screening can be carried out by use of methods known in the art (see e.g., Omasa T.: J. Biosci. Bioeng., 94, 600-605, 2002).

The antibody of the present invention includes an antibody composed of two heavy chains and two light chains as well as an antigen-binding fragment of the antibody. The antigen-binding fragment includes, for example, Fab (fragment of antigen binding), Fab', F(ab')2, and single chain Fv (scFv) and disulfide stabilized Fv (dsFv) as active fragments of the antibody linked via a linker or the like. Examples of peptides comprising the active fragments of the antibody include peptides containing CDRs. These fragments can be produced by a method known in the art such as a method of treating the antibody of the present invention with an appropriate protease or a gene recombination technique.

The antibody can be purified by use of purification means known in the art such as salting out, gel filtration, ion-exchange chromatography, or affinity chromatography.

Alternatively, according to a recently developed phage display antibody technique which involves expressing a recombinant antibody on phage surface by use of a genetic engineering technique, human VH and VL genes may be artificially shuffled, and diversified scFv (single chain fragment of variable region) antibodies can be expressed as phage-fused proteins to obtain specific antibodies. Any specific antibody or antigen-binding fragment thereof prepared by use of this technique with reference to the amino acid sequences of SEQ ID NOs: 2 to 6, 8 to 12, 14 to 18, and 20 to 24 described herein is included in the technical scope of the present invention. In addition, a recombinant antibody having high neutralizing activity or excellent thermal stability modified by the application of a humanized antibody preparation technique on the basis of the CDR sequence information described above (see e.g., WO2007/139164) is also included in the scope of the present invention.

Alternatively, an antibody or the like prepared by a recently developed technique of drastically improving the ADCC activity of an antibody by the modification of the sugar chain moiety of the antibody, for example, an antibody obtained by the application of Potelligent technique to the antibody of the present invention (see Niwa R., et al, Clin. Cancer Res., 10, 6248-6255 (2004)), and an antibody or the like prepared by a technique of improving CDC activity, for example, an antibody obtained by the application of Complegent technique to the antibody of the present invention (see Kanda S., et al, Glycobiology, 17, 104-118 (2007)) are also included in the technical scope of the present invention. In addition, an antibody obtained by an antibody modification technique intended to maintain the *in vivo* concentration of the antibody by the partial substitution of an Fc region (see e.g., WO2007/114319) is also included in the technical scope of the present invention.

Furthermore, any antibody or antigen-binding fragment thereof obtained by the application of a partial Fc region substitution technique (see WO2006/071877) performed in order to impart protease resistance ability to the antibody and renders the antibody orally administrable is included in the technical scope of the present invention.

As an antibody preparation approach, a polyclonal antibody or a monoclonal antibody is usually obtained by use of a laboratory animal such as a mouse, a rabbit, or a goat. Since the antibody thus obtained has a sequence characteristic of the animal species used, the antibody administered directly to a human may be recognized as foreign matter by the human immune system to cause human anti-animal antibody response (i.e., to yield an antibody against the antibody).

The anti-*C*. *difficile* toxin monoclonal antibody of the present invention or the antigen-binding fragment thereof can be obtained from antibody-producing cells derived from the blood of a healthy person or the like. In this case, the antibody is a completely human antibody. This completely human antibody probably has low immunogenicity even if administered as an antibody drug to a human body.

Particularly, the anti-*C*. *difficile* toxin A monoclonal antibody of the present invention has higher neutralizing ability than that of the conventional anti-*C*. *difficile* toxin A monoclonal antibodies and as such, can be expected to produce equivalent therapeutic effects at a lower dose.

### 5. Pharmaceutical composition containing antibody of the present invention

The present invention further provides a pharmaceutical composition for the prevention or treatment of a disease involving a *C. difficile* toxin, comprising the antibody or the antigen-binding site thereof and a pharmaceutically acceptable carrier.

Particularly, the anti-*C*. *difficile* toxin A antibody of the present invention or the antigen-binding fragment thereof has higher neutralizing ability against toxin A than that of the conventional anti-toxin A antibodies and as such, is useful as a preventive or therapeutic drug for a disease involving the C. *difficile* toxin. As shown in Example 13, the neutralizing antibody against toxin A can be used not only alone but in combination with the neutralizing antibody against toxin B in the treatment of CDI. The combined administration can further enhance preventive and therapeutic effects on CDI compared with the administration of each antibody alone. Specifically, the combined administration of a pharmaceutical composition comprising any one antibody selected from among the anti-toxin A antibodies of the present invention and the antigen-binding fragments thereof or a pharmaceutical composition comprising a plurality of antibodies selected from thereamong, and a pharmaceutical composition comprising any one antibody selected from among the anti-toxin B antibodies of the present invention and the antigen-binding fragments thereof or a pharmaceutical composition comprising a plurality of antibodies selected from thereamong can be expected to produce a remarkable protective effect against CDI.

The "pharmaceutically acceptable carrier" used herein includes any or every biologically compatible solvent, dispersion, coating, tonicity agent, and absorption-delaying agent, etc.

Examples of the pharmaceutically acceptable carrier include one or more carriers such as water, a salt solution, phosphate-buffered saline, dextrose, glycerol, and ethanol, and combinations thereof. For use as an injection or the like, the composition preferably contains a pH adjuster or a tonicity agent, for example, a sugar, a polyalcohol (mannitol, sorbitol, etc.), or sodium chloride. The pharmaceutically acceptable carrier can further include a small amount of an auxiliary substance that enhances the conservation or effectiveness of the antibody or a moiety of the antibody, such as a wetting agent, an emulsifier, an antiseptic, a buffer, or a stabilizer.

The composition of the present invention can be prepared in various dosage forms. Such a composition includes liquid, semisolid, and solid dosage forms, for example, solutions (e.g., injectable and transfusable solutions), dispersions, suspensions, tablets, capsules, troches, pills, powders, liposomes, and suppositories. Preferred forms differ depending on the intended mode of administration and a case to which treatment is applied. The composition is generally preferably in the form of an injectable or transfusable solution, as with, for example, compositions similar to those used for the passive immunization of humans with other antibodies. The preferred mode of administration is parenteral administration (e.g., intravenous, subcutaneous, intraperitoneal, or intramuscular administration). In a preferred embodiment, the antibody is administered through intravenous transfusion or intravenous injection. In another preferred embodiment, the antibody is administered through intramuscular injection or subcutaneous injection.

The antibody of the present invention or the antibody fragment can be incorporated into a pharmaceutical composition suitable for parenteral administration. For example, when the antibody or the antibody fragment is prepared as an injectable preparation, the concentration range of the preparation is preferably 0.1 to 200 mg/mL, more preferably 1 to 120 mg/mL, further more preferably 2 to 25 mg/mL.

Exemplary injectable preparations will be described below. However, the preparation of the present invention is not limited thereto as long as the preparation is preferred as an injection of the antibody drug of the present invention. The preparation can be constituted by, for example, a flint or amber vial, an ampule, or a prefilled syringe containing an active ingredient dissolved in a liquid or a freeze-dried active ingredient. The buffer can be L-histidine (1 to 50 mM) having a pH of 5.0 to 7.0 (optimally pH 6.0), optimally 5 to 10 mM L-histidine. Other appropriate buffers include, but are not limited to, sodium succinate, sodium citrate, sodium phosphate, and potassium phosphate. Sodium chloride can be used in order to change the osmotic pressure of a solution having a concentration of 0 to 300 mM (optimally 150 mM for the liquid dosage form,). The freeze-dried dosage form can contain a cryoprotectant, mainly, 0 to 10% (optimally 0.5 to 5.0%) sucrose. Other appropriate cryoprotectants include mannitol, trehalose, and lactose. The freeze-dried dosage form can contain an expander, mainly, 1 to 10% (optimally 2 to 4%) mannitol. In both of the liquid and freeze-dried dosage forms, a stabilizer, mainly, 1 to 50 mM (optimally 5 to 10 mM) L-methionine can be used. Other appropriate stabilizers include glycine, arginine, and polysorbate 80, etc. In the case of polysorbate 80, 0 to 0.05% (optimally 0.005 to 0.01%) can be contained therein. Other surfactants include, but are not limited to, polysorbate 20 and BRIJ surfactants.

In general, the pharmaceutical composition of the present invention must be sterile or stable under production and preservation conditions. This composition can be formulated as a solution, a microemulsion, a dispersion, a liposome, or other ordered structures suitable for high drug concentrations. The sterile injectable solution can be prepared by mixing a necessary amount of an active compound (i.e., the antibody or a moiety of the antibody), if necessary together with one or combination of the aforementioned components, into an appropriate solvent, followed by filtration sterilization. In general, the active compound is mixed with a sterile vehicle containing a basic dispersion medium and other necessary components selected from those listed above to prepare a dispersion. Preferred methods for preparing a sterile powder preparation for preparing the sterile injectable solution are the vacuum freeze drying and spray drying of the sterile filtrate thereof mentioned above. As a result, a composition comprising a powder of the active ingredient as well as arbitrary other desired components is obtained. The adequate flowability of the solution can be maintained, for example, by using coating with lecithin or the like, by maintaining a necessary particle size in the case of the dispersion, or by using a surfactant. The long-term absorption of the injectable composition can be achieved by means of absorption-delaying agent, for example, monostearate or gelatin, contained in the composition.

The present invention also relates to a kit comprising the anti-toxin antibody of the present invention or the antigen-binding site thereof. The kit may contain, for example, one or more additional components including other substances (pharmaceutically acceptable carriers) listed herein for preparing the pharmaceutical composition of the antibody, and an apparatus or other substances for administration to a subject. In this case, various combinations of pharmaceutical compositions of individual antibodies can be packaged together. The pharmaceutical compositions contained in the kit are constituted by, for example, pharmaceutical compositions respectively comprising any one or more selected from among the first monoclonal antibodies neutralizing toxin A or the antigen-binding fragments thereof, and any one or more selected from among the second monoclonal antibodies neutralizing toxin B or the antigen-binding fragments thereof. Preferably, the first antibody is EV029105a or an anti-toxin A antibody within the scope of the present invention having neutralizing activity equivalent to or higher than that of EV029105a, and the second antibody is EV029104 or an anti-toxin B antibody within the scope of the present invention having neutralizing activity equivalent to or higher than that of EV029104. Particularly, plural types of antibodies may be mixed, and the resulting preparation can be packaged in the kit as long as there is no problem associated with stability.

### 6. Approach of obtaining anti-C. difficile toxin antibody of the present invention and antigen-binding fragment thereof

Next, the approaches by which the anti-C. *difficile* toxin monoclonal antibody of the present invention and the antigen-binding fragment thereof were obtained will be described. However, the approaches of obtaining the antibody of the present invention, etc., are not limited by the description below. As mentioned above, changes or modifications usually performed in the art can be made therein, as a matter of course.

The anti-C. *difficile* toxin antibody of the present invention and the antigen-binding fragment thereof can be obtained by: separating a cell clone producing the antibody through various steps from the blood of a human; isolating cDNA from the obtained cell clone and amplifying the cDNA; transferring plasmids having the cDNA incorporated therein to producing cells; and culturing the obtained antibody-producing cells, followed by, for example, affinity purification from the supernatant.

### 1) Separation of cell clone producing completely human antibody against C. difficile toxin

B lymphocytes are separated from the blood of a human, and the growth of the B lymphocytes is induced. The method for inducing the growth is known in the art per se and can be carried out by, for example, a transformation method (D. Kozbor et al.) using "Epstein-Barr virus (EB virus)" (hereinafter, referred to as EBV), which is a factor triggering cancer.

Specifically, the B lymphocytes are infected by EBV to induce its growth. The cells that have grown are used as an antibody-producing cell library.

### 2) Recovery of monoclonal antibody from antibody-producing cell library

The method for recovering monoclonal antibodies from the cells that have grown by induction can be carried out by a well known method routinely used in the preparation of monoclonal antibodies.

The antibody-producing cell library is screened for a lymphocyte clone producing an antibody binding to *C. difficile* toxin A or/and *C. difficile* toxin B. From the obtained cell clone, cDNA is isolated and amplified. Plasmids having an insert of the cDNA are transferred to producing cells. The obtained antibody-producing cells are cultured, and the antibody is isolated from the culture supernatant. In order to separate the cell clone of interest from the antibody-producing cell library, a cell population (clone) producing the antibody binding to the *C. difficile* toxin can be selected by the appropriate combination of a limiting dilution culture method, a sorting method, and a cell microarray method.

The clone binding to the C. *difficile* toxin is preferably detected by use of ELISA with the *C. difficile* toxin as an antigen and ELISA using a labeled mouse anti-human IgG antibody.

The selected antibody-positive cell population can be cultured and repeatedly screened to obtain a cell population (clone) producing only the antibody of interest.

A flowchart representing these steps up to the separation of the antibody-producing cell clone is shown in Figure 1.

### 3) Affinity purification using protein A or G

For the purification of the anti-*C*. *difficile* toxin antibody, antibody-producing cells can be obtained by a gene recombination approach from the selected cells and allowed to grow in a roller bottle, a 2-L spinner flask, or a different culture system.

The obtained culture supernatant can be filtered, concentrated, and then subjected to affinity chromatography using protein A or protein G-Sepharose (GE Healthcare Japan Corp.) or the like to purify the protein. The buffer solution is replaced with PBS, and the concentration can be determined by OD280 or, preferably, nephelometer analysis. The isotype can be examined by a method specific for the isotype antigen. The anti-*C*. *difficile* toxin antibody thus obtained is a completely human antibody prepared from B lymphocytes sensitized in the human body and is therefore substantially unlike to cause immune response.

Another feature of this approach is that the EB virus having the activity of inducing the growth of B lymphocytes by infection is used in the preparation of the antibody-producing cell clone.

The EB virus method has the advantages that: a natural human antibody produced in a human body can be prepared; and an antibody having high affinity can be obtained. For example, a human antibody against a certain kind of virus (e.g., human CMV) has been found to have approximately 10 to 100 times higher affinity than that of an antibody prepared from an artificially immunized mouse. The B lymphocyte population that has grown by EB virus infection serves as a library of antibody-producing cells. From this library, a particular antibody-producing cell clone is separated, and antibody-producing cells can be obtained by a gene recombination method from the separated and selected cells and cultured to obtain a human antibody.

### EXAMPLES

Hereinafter, the present invention will be described further specifically with reference to Examples. However, the present invention is not intended to be limited by these Examples by any means. For the procedures used in these Examples, see Molecular Cloning: A Laboratory Manual (Third Edition) (Sambrook et al., Cold Spring Harbour Laboratory Press, 2001), unless otherwise specified.

### Example 1. Separation of cell clone producing completely human antibody against C. difficile toxin A or toxin B

A typical flowchart for the separation of an antibody-producing cell clone is shown in Figure 1.

B lymphocytes were separated from anti-C. *difficile* toxin antibody-positive human peripheral blood and infected by EBV. The infected cells were inoculated to a 96-well plate, cultured for 3 to 4 weeks, and then screened for anti-C. *difficile* toxin antibodies in the culture supernatant. The screening was carried out by ELISA targeting antibodies against toxin A (SEQ ID NO: 25; GenBank Accession No. P16154) and toxin B (SEQ ID NO: 26; GenBank Accession No. Q46034) (Clin. Microbiol. Rev., 18, 247-263 (2005); and GlycoBiol., 17, 15-22 (2007)), which are primary exotoxins of C. *difficile,* and using a 96-well plate coated with toxin A or toxin B (both obtained from List Biological Laboratories Inc.). The cells in each well confirmed to contain the produced anti-C. *difficile* toxin antibodies were subjected to a limiting dilution culture method appropriately combined with a sorting method and a cell microarray method to separate a cell clone producing each antibody of interest.

### Example 2. Confirmation of antibody isotype and subclass

Each produced antibody was isotyped by ELISA using the culture supernatant of the separated antibody-producing cell clone (see reference: Curr Protoc Immunol. 2001 May; Chapter 2: Unit 2.2). This ELISA employed a 96-well plate coated with toxin A or toxin B to which each anti-toxin antibody was allowed to bind. Next, an antibody specific for each isotype and subclass was used as a secondary antibody. The isotype and subclass of the obtained anti-toxin A antibody or anti-toxin B antibody is shown in Table 1.

**[Table 1]**

| Antibody No. | Target antigen | Subclass |
|---|---|---|
| EV029105a | Toxin A | IgG1/κ |
| EV029104 | Toxin B | IgG1/λ |

### Example 3. Cloning of cDNA encoding anti-C. difficile toxin antibody

The total RNA of the antibody-producing cells was reverse-transcribed using oligo-dT primers. The obtained cDNA was used as a template in the PCR amplification of each antibody gene. The primers used in PCR were designed on the basis of the database of cDNAs encoding human IgG antibody H and L chains. In order to amplify the full-length H chain cDNA and L chain cDNA, the 5' primer has a translation initiation point, and the 3' primer has a translation termination point.

### Example 4. Determination of amino acid sequence of antibody based on nucleotide sequence

The H chain and L chain cDNAs of each antibody amplified by PCR were inserted to plasmid vectors, and their nucleotide sequences were confirmed using an ABI sequencer. The signal sequence, H chain and L chain amino acid sequences, variable region amino acid sequences, and complementarity-determining region (CDR) amino acid sequences of the antibody were each determined from the obtained nucleotide sequences. In the CDR analysis, the method of Kabat (www.bioinf.org.uk: Dr. Andrew C.R. Martin's Group, Antibodies: General Information) was used.

SEQ ID NO of each sequence is described below.
SEQ ID NO: 1: the nucleotide sequence of the H chain (including the signal sequence) of EV029105a
SEQ ID NO: 2: the amino acid sequence of the H chain (including the signal sequence) of EV029105a
SEQ ID NO: 3: the amino acid sequence of the H chain variable region of EV029105a
SEQ ID NO: 4: the amino acid sequence of the H chain CDR1 region of EV029105a
SEQ ID NO: 5: the amino acid sequence of the H chain CDR2 region of EV029105a
SEQ ID NO: 6: the amino acid sequence of the H chain CDR3 region of EV029105a
SEQ ID NO: 7: the nucleotide sequence of the L chain (including the signal sequence) of EV029105a
SEQ ID NO: 8: the amino acid sequence of the L chain (including the signal sequence) of EV029105a
SEQ ID NO: 9: the amino acid sequence of the L chain variable region of EV029105a
SEQ ID NO: 10: the amino acid sequence of the L chain CDR1 region of EV029105a
SEQ ID NO: 11: the amino acid sequence of the L chain CDR2 region of EV029105a
SEQ ID NO: 12: the amino acid sequence of the L chain CDR3 region of EV029105a
SEQ ID NO: 13: the nucleotide sequence of the H chain (including the signal sequence) of EV029104
SEQ ID NO: 14: the amino acid sequence of the H chain (including the signal sequence) of EV029104
SEQ ID NO: 15: the amino acid sequence of the H chain variable region of EV029104
SEQ ID NO: 16: the amino acid sequence of the H chain CDR1 region of EV029104
SEQ ID NO: 17: the amino acid sequence of the H chain CDR2 region of EV029104
SEQ ID NO: 18: the amino acid sequence of the H chain CDR3 region of EV029104
SEQ ID NO: 19: the nucleotide sequence of the L chain (including the signal sequence) of EV029104
SEQ ID NO: 20: the amino acid sequence of the L chain (including the signal sequence) of EV029104
SEQ ID NO: 21: the amino acid sequence of the L chain variable region of EV029104
SEQ ID NO: 22: the amino acid sequence of the L chain CDR1 region of EV029104
SEQ ID NO: 23: the amino acid sequence of the L chain CDR2 region of EV029104
SEQ ID NO: 24: the amino acid sequence of the L chain CDR3 region of EV029104

### Example 5. Confirmation that obtained antibody gene encoding anti-C. difficile toxin antibody

The obtained H chain and L chain cDNAs were respectively inserted to expression vectors. CHO cells were cotransfected with the expression vectors for transient expression. The transfection was carried out using Lipofectamine (Invitrogen Corp.) and Plus reagent (Invitrogen Corp.) under conditions recommended by the manufacturer (Invitrogen catalog: Cat. No. 18324-111, Cat. No. 18324-012, or Cat. No. 18324-020). Two days later, the culture supernatant was recovered. By ELISA using an anti-human IgG antibody and a 96-well plate coated with toxin A or toxin B, it was confirmed that: the antibodies in the culture supernatants of EV029105a and EV029104 were human IgG antibodies; and EV029105a and EV029104 specifically bound to toxin A and toxin B, respectively.

### Example 6. Production of antibody protein

CHO cells were transfected with the expression plasmid for each obtained anti-C. *difficile* toxin antibody. The transfection was carried out in the same way as above. The cells were cultured in the presence of a selection marker to obtain a CHO cell clone constitutively producing each antibody.

The CHO cells stably producing each antibody were cultured in a serum-free medium, and each culture supernatant was recovered. This culture supernatant was added to a protein A column and subjected to affinity purification to obtain a purified antibody. The column used was a prepack column of HiTrap rProtein A FF (GE Healthcare Japan Corp.). The purification conditions were set to conditions recommended by the column manufacturer. After the purification, the binding activity of each antibody against toxin A or toxin B was confirmed by ELISA. Also, the presence of an antibody H chain of approximately 50 kDa and an antibody L chain of approximately 25 kDa was confirmed in each antibody by SDS-PAGE.

### Example 7. Neutralizing activity of anti-toxin A antibody

Each antibody was tested for its neutralizing activity against toxin A *in vitro* (Babcock et al., Infect. Immun. 74: 6339-6347 (2006)). Toxin A was reacted in the presence of varying concentrations of the toxin A-specific monoclonal antibody and then added to cells to evaluate the ability to block the rounding of the toxin A-exposed cells. Human lung fibroblasts IMR-90 were used as the target cells of toxin A. For the activity evaluation, the rate of rounding (%) was determined by the visual observation of the cells and indicated as a cytopathic effect (CPE). The anti-toxin A antibodies used in this activity evaluation were EV029105a and two already publicly available anti-toxin A antibodies 3D8 (WO2006/121422) and hPA-50 (WO2011/130650). The antibodies 3D8 and hPA-50 were both prepared on the basis of their reported nucleotide sequences. A human monoclonal antibody (EV2037; WO2010/114105) specific for human cytomegalovirus (HCMV) was used as a negative control. The results of these experiments are shown in Figures 2A to 2D. These diagrams showed that all of the antibodies except for EV2037 suppress the rounding of cells by toxin A. The neutralizing activity (EC50 value) of these anti-toxin A antibodies against the cytotoxicity of toxin A to the IMR-90 cells is shown in Table 2. Among these three anti-toxin A antibodies, an antibody having high neutralizing activity of 100 ng/mL or lower was only EV029105a.

**[Table 2]**

| Antibody No. | EC50 (ng/mL) |
|---|---|
| EV029105a | 4.92 ± 0.78 (0.033nM) |
| 3D8 | 219.1 ± 26.7 |
| hPA-50 | 4970 ± 2880 |

### Example 8. Neutralizing activity of anti-toxin B antibody

Each antibody was tested for its neutralizing activity against toxin B *in vitro* (Babcock et al., Infect. Immun. 74: 6339-6347 (2006)). Toxin B was reacted in the presence of varying concentrations of the toxin B-specific monoclonal antibody and then added to cells to evaluate the ability to block the rounding of the toxin B-exposed cells. Human lung fibroblasts IMR-90 were used as the target cells of toxin B. For the activity evaluation, the rate of rounding (%) was determined by the visual observation of the cells and indicated as a cytopathic effect (CPE). The anti-toxin B antibodies used in this activity evaluation were EV029104 and two already publicly available anti-toxin B antibodies MDX1388 (WO2006/121422) and hPA-41 (WO2011/130650). The antibodies MDX1388 and hPA-41 were both prepared on the basis of their reported nucleotide sequences. A human monoclonal antibody (EV2037; WO2010/114105) specific for human cytomegalovirus (HCMV) was used as a negative control. These experiments showed that all of EV029104, MDX1388, and hPA-41 have neutralizing activity (EC50 value) of 100 ng/mL or lower.

### Example 9. Affinity of anti-toxin A antibody

The affinity of each antibody for toxin A was measured using a Biacore T200(R) apparatus for detecting the binding interaction between biomolecules by surface plasmon resonance. To a sensor chip CAP, biotinylated toxin A was added, and each antibody was flowed on the chip to measure its binding activity. EV029105a had K_{D} of 1.51 X 10⁻¹⁰ M. This binding activity was compared with that of the anti-toxin A antibodies 3D8 and hPA-50 measured in the same way as above. The results are shown in Table 3 below. These results demonstrated that among these three anti-toxin A antibodies, EV029105a has the highest affinity.

**[Table 3]**

| Antibody No. | Kₐ (1/Ms) | K_{d} (1/s) | K_{D} (M) |
|---|---|---|---|
| EV029105a | 6.53 × 10⁵ | 9.89 × 10⁻⁵ | 1.51 × 10⁻¹⁰ |
| 3D8 | 5.87 × 10⁵ | 4.22 × 10⁻⁴ | 7.19 × 10⁻¹⁰ |
| hPA-50 | 8.31 × 10⁵ | 3.49 × 10⁻⁴ | 4.20 × 10⁻¹⁰ |

### Example 10. Epitope mapping of anti-toxin A antibody

An epitope on toxin A (SEQ ID NO: 25, GenBank Accession No. P16154) to which each antibody bound was determined by Western blotting. A recombinant E. *coli* clone expressing four fragments of toxin A, i.e., the enzyme domain (i.e., amino acids 1 to 659 of toxin A), the receptor-binding domain (i.e., amino acids 1853 to 2710 of toxin A), and two intermediate regions (i.e., amino acids 660 to 1256 and 1257 to 1852 of toxin A), was constructed. Each moiety of toxin A (SEQ ID NO: 25) was amplified by PCR from C. *difficile* 630 strain-derived genomic DNA. These fragments were cloned using pGEX vectors, and BL21 DE3 cells were transformed with the vectors. The expression was induced using IPTG, and Western blotting analysis was conducted with cell lysates of the four fragments of toxin A as antigens. Fragment 1 was the moiety of amino acids 1 to 659; fragment 2 was the moiety of amino acids 660 to 1256; fragment 3 was the moiety of amino acids 1257 to 1852; and fragment 4 was the moiety of amino acids 1853 to 2710. All of EV029105a, 3D8, and hPA-50 reacted with the fragment 4 (receptor-binding domain). In order to find out whether or not EV029105a, 3D8, and hPA-50 bound to the same epitope on the receptor-binding domain, competition assay was conducted using a Biacore T200(R) apparatus. To a sensor chip CAP, biotinylated toxin A was added, subsequently the EV029105a antibody was added, and finally the 3D8 or hPA-50 antibody was added. The binding of 3D8 and hPA-50 to toxin A was not inhibited, even though EV029105a was first bound with toxin A. These results demonstrated that EV029105a binds to an epitope different from that for 3D8 and hPA-50. These results are shown in Figures 3A to 3C.

### Example 11. Epitope mapping of anti-toxin B antibody

An epitope on toxin B (SEQ ID NO: 26, GenBank Accession No. Q46034) to which each antibody bound was determined by Western blotting. A recombinant E. *coli* clone expressing four fragments of toxin B, i.e., the enzyme domain (i.e., amino acids 1 to 546 of toxin B), the receptor-binding domain (i.e., amino acids 1777 to 2366 of toxin B), and two intermediate regions (i.e., amino acids 547 to 1184 and 1185 to 1776 of toxin B), was constructed. Each moiety of toxin B (SEQ ID NO: 26) was amplified by PCR from C. *difficile* 630 strain-derived genomic DNA. These fragments were cloned using pGEX vectors, and BL21 DE3 cells were transformed with the vectors. The expression was induced using IPTG, and Western blotting analysis was conducted with cell lysates of the four fragments of toxin B as antigens. Fragment 1 was the moiety of amino acids 1 to 546; fragment 2 was the moiety of amino acids 547 to 1184; fragment 3 was the moiety of amino acids 1185 to 1776; and fragment 4 was the moiety of amino acids 1777 to 2366. EV029104 and MDX1388 reacted with the fragment 4 (receptor-binding domain), while hPA-41 reacted with the fragment 1 (enzyme domain). This showed the toxin B-binding site of hPA-41 is different from that of EV029104.

In order to further find out whether or not EV029104 and MDX1388 bound to the same epitope on the receptor-binding domain, competition assay was conducted using a Biacore T200(R) apparatus. To a sensor chip CAP, biotinylated toxin B was added, subsequently the EV029104 antibody was added, and finally the MDX1388 antibody was added. The binding of MDX1388 to toxin B was not inhibited, even though EV029104 was first bound with the toxin. These results demonstrated that EV029104 binds to an epitope on toxin B different from that for MDX1388. These results are shown in Figure 4.

### Example 12. Protective effect of anti-toxin A antibody against mouse lethality by toxin A

A test was conducted on whether or not each antibody was able to protect a mouse against the lethal effect of toxin A (WO2006/121422 and WO2011/130650). Each antibody or a control antibody (anti-cytomegalovirus antibody) was intraperitoneally administered (0.165 to 50 µg/head) to 4-week-old Swiss Webster female mice (10 to 15 individuals per group). Approximately 24 hours after the antibody administration, 200 ng of toxin A was intraperitoneally inoculated to each mouse. Approximately 24 hours thereafter, the sign of toxicity was observed and determined on the basis of the survival rate of each animal. The results of these experiments are shown in Figure 5. For example, EV029105a was confirmed to have a protective effect of 50% even in the administration group that received a low dose of 0.165 µg/head. In the comparison of each anti-toxin A antibody in the 0.5 µg/head administration group, the administration of 3D8 or hPA-50 exhibited a protective effect of 50%, whereas a protective effect as high as 90% was observed in the EV029105a administration group. This *in vivo* neutralization test showed that EV029105a has high neutralizing activity compared with 3D8 or hPA-50.

### Example 13. Protective effects of anti-toxin A antibody and anti-toxin B antibody against lethality of Syrian golden hamster by C. difficile

Ten Syrian golden hamsters (5-week-old male, approximately 80 g) were used per group. *C. difficile* (545 strain, ATCC) was orally administered at 100 spores/hamster using a gastric tube. On the day before C. *difficile* inoculation, 10 mg/kg clindamycin (Nipro Pharma Corp.) was intraperitoneally administered in order to enhance sensitivity to infection. The anti-toxin A antibody EV029105a and the anti-toxin B antibody EV029104 were intraperitoneally administered a total of three times (the day before C. *difficile* inoculation, the C. *difficile* inoculation day, and the next day). Results of showing the survival rates of the hamsters in each group are shown in Figures 6A to 6B.

Figure 6A shows the results of comparing antibody administration groups with a group given only clindamycin without the administration of the antibody, wherein the antibody administration groups were set to two groups: a group given EV029105a and EV029104 both at 50 mg/kg per dose; and a group given EV029105a at 10 mg/kg and EV029104 at 50 mg/kg. All of the hamsters in the group without the administration of the antibody died 2 days after the C. *difficile* inoculation. By contrast, all of the hamsters in the group given EV029105a and EV029104 both at 50 mg/kg survived up to 10 days after the C. *difficile* inoculation, and all of the hamsters in the group given EV029105a at 10 mg/kg and EV029104 at 50 mg/kg survived up to 5 days after the C. *difficile* inoculation.

Figure 6B shows the results of comparing antibody administration groups with a group given only clindamycin without the administration of the antibody, wherein the dose of the antibody was decreased and the antibody administration groups were set to four groups: a group given EV029105a at 10 mg/kg and EV029104 at 10 mg/kg per dose; a group given EV029105a at 10 mg/kg and EV029104 at 2 mg/kg; a group given only EV029105a at 10 mg/kg; and a group given only EV029105a at 2 mg/kg. All of the hamsters in the group without the administration of the antibody died 2 days after the *C. difficile* inoculation. By contrast, as for the antibody administration groups, the groups that received the combined administration of EV029105a and EV029104 exhibited an EV029104 dose-dependent rise in survival rate and 90% of the hamsters in the group given EV029105a at 10 mg/kg and EV029104 at 10 mg/kg survived even 4 days after the *C. difficile* inoculation, though all the hamsters in the group given only EV029105a died within 4 days after the *C. difficile* inoculation. These results demonstrated that the combined administration of EV029105a and EV029104 remarkably protects the hamsters in a dose-dependent manner against the lethal effect of C. *difficile.*

### INDUSTRIAL APPLICABILITY

The monoclonal antibody of the present invention is useful as a medicine for the treatment of *Clostridium difficile* infection.

## Claims

1. An antibody capable of specifically binding to *Clostridium difficile* toxin A protein and neutralizing its biological activity, or an antigen-binding fragment thereof, comprising:
(i)
(a) heavy chain CDR1 comprising the amino acid sequence of SEQ ID NO: 4 or an amino acid sequence deviated from the amino acid sequence of SEQ ID NO: 4 by deletion, substitution, insertion, and/or addition mutations of one to several amino acid residues,
(b) heavy chain CDR2 comprising the amino acid sequence of SEQ ID NO: 5 or an amino acid sequence deviated from the amino acid sequence of SEQ ID NO: 5 by deletion, substitution, insertion, and/or addition mutations of one to several amino acid residues, and
(c) heavy chain CDR3 comprising the amino acid sequence of SEQ ID NO: 6 or an amino acid sequence deviated from the amino acid sequence of SEQ ID NO: 6 by deletion, substitution, insertion, and/or addition mutations of one to several amino acid residues; and
(ii)
(a) light chain CDR1 comprising the amino acid sequence of SEQ ID NO: 10 or an amino acid sequence deviated from the amino acid sequence of SEQ ID NO: 10 by deletion, substitution, insertion, and/or addition mutations of one to several amino acid residues,
(b) light chain CDR2 comprising the amino acid sequence of SEQ ID NO: 11 or an amino acid sequence deviated from the amino acid sequence of SEQ ID NO: 11 by deletion, substitution, insertion, and/or addition mutations of one to several amino acid residues, and
(c) light chain CDR3 comprising the amino acid sequence of SEQ ID NO: 12 or an amino acid sequence deviated from the amino acid sequence of SEQ ID NO: 12 by deletion, substitution, insertion, and/or addition mutations of one to several amino acid residues.

2. The antibody or an antigen-binding fragment thereof according to claim 1, comprising:
(i)
(a) the amino acid sequence of heavy chain CDR1 comprising the amino acid sequence of SEQ ID NO: 4,
(b) the amino acid sequence of heavy chain CDR2 comprising the amino acid sequence of SEQ ID NO: 5, and
(c) the amino acid sequence of heavy chain CDR3 comprising the amino acid sequence of SEQ ID NO: 6; and
(ii)
(a) the amino acid sequence of light chain CDR1 comprising the amino acid sequence of SEQ ID NO: 10,
(b) the amino acid sequence of light chain CDR2 comprising the amino acid sequence of SEQ ID NO: 11, and
(c) the amino acid sequence of light chain CDR3 comprising the amino acid sequence of SEQ ID NO: 12.

3. The antibody or an antigen-binding fragment thereof according to claim 1 or 2, comprising
(a) a heavy chain variable region (VH) comprising the amino acid sequence of SEQ ID NO: 3 or an amino acid sequence having 95% or higher identity to the amino acid sequence of SEQ ID NO: 3, and
(b) a light chain variable region (VL) comprising the amino acid sequence of SEQ ID NO: 9 or an amino acid sequence having 95% or higher identity to the amino acid sequence of SEQ ID NO: 9.

4. The antibody or an antigen-binding fragment thereof according to any one of claims 1 to 3, comprising
(a) a heavy chain variable region (VH) comprising the amino acid sequence of SEQ ID NO: 3, and
(b) a light chain variable region (VL) comprising the amino acid sequence of SEQ ID NO: 9.

5. The antibody or an antigen-binding fragment thereof according to any one of claims 1 to 4, wherein the antibody or the antigen-binding fragment thereof is of IgG1 (κ) class (subclass).

6. The antibody or an antigen-binding fragment thereof according to any one of claims 1 to 5, wherein the antibody or the antigen-binding fragment thereof has a dissociation constant (K_{D} value) of 1 x 10⁻⁹ M or lower against the *Clostridium difficile* toxin A.

7. The antibody or an antigen-binding fragment thereof according to any one of claims 1 to 5, wherein the antibody or the antigen-binding fragment thereof has a neutralizing activity (EC50) of 0.05 µg/mL (approximately 0.33 nM) or lower against the *Clostridium difficile* toxin A in measurement using a human lung fibroblast IMR-90.

8. An antibody capable of specifically binding to *Clostridium difficile* toxin B protein and neutralizing its biological activity, or an antigen-binding fragment thereof, containing:
(i)
(a) heavy chain CDR1 comprising the amino acid sequence of SEQ ID NO: 16 or an amino acid sequence deviated from the amino acid sequence of SEQ ID NO: 16 by deletion, substitution, insertion, and/or addition mutations of one to several amino acid residues,
(b) heavy chain CDR2 comprising the amino acid sequence of SEQ ID NO: 17 or an amino acid sequence deviated from the amino acid sequence of SEQ ID NO: 17 by deletion, substitution, insertion, and/or addition mutations of one to several amino acid residues, and
(c) heavy chain CDR3 comprising the amino acid sequence of SEQ ID NO: 18 or an amino acid sequence deviated from the amino acid sequence of SEQ ID NO: 18 by deletion, substitution, insertion, and/or addition mutations of one to several amino acid residues; and
(ii)
(a) light chain CDR1 comprising the amino acid sequence of SEQ ID NO: 22 or an amino acid sequence deviated from the amino acid sequence of SEQ ID NO: 22 by deletion, substitution, insertion, and/or addition mutations of one to several amino acid residues,
(b) light chain CDR2 comprising the amino acid sequence of SEQ ID NO: 23 or an amino acid sequence deviated from the amino acid sequence of SEQ ID NO: 23 by deletion, substitution, insertion, and/or addition mutations of one to several amino acid residues, and
(c) light chain CDR3 comprising the amino acid sequence of SEQ ID NO: 24 or an amino acid sequence deviated from the amino acid sequence of SEQ ID NO: 24 by deletion, substitution, insertion, and/or addition mutations of one to several amino acid residues.

9. The antibody or the antigen-binding fragment thereof according to claim 8, comprising:
(i)
(a) the amino acid sequence of heavy chain CDR1 comprising the amino acid sequence of SEQ ID NO: 16,
(b) the amino acid sequence of heavy chain CDR2 comprising the amino acid sequence of SEQ ID NO: 17, and
(c) the amino acid sequence of heavy chain CDR3 comprising the amino acid sequence of SEQ ID NO: 18; and
(ii)
(a) the amino acid sequence of light chain CDR1 comprising the amino acid sequence of SEQ ID NO: 22,
(b) the amino acid sequence of light chain CDR2 comprising the amino acid sequence of SEQ ID NO: 23, and
(c) the amino acid sequence of light chain CDR3 comprising the amino acid sequence of SEQ ID NO: 24.

10. The antibody or the antigen-binding fragment thereof according to claim 8 or 9, comprising
(a) a heavy chain variable region (VH) comprising the amino acid sequence of SEQ ID NO: 15 or an amino acid sequence having 95% or higher identity to the amino acid sequence of SEQ ID NO: 15, and
(b) a light chain variable region (VL) comprising the amino acid sequence of SEQ ID NO: 21 or an amino acid sequence having 95% or higher identity to the amino acid sequence of SEQ ID NO: 21.

11. The antibody or the antigen-binding fragment thereof according to any one of claims 8 to 10, comprising
(a) a heavy chain variable region (VH) comprising the amino acid sequence of SEQ ID NO: 15, and
(b) a light chain variable region (VL) comprising the amino acid sequence of SEQ ID NO:21.

12. The antibody or the antigen-binding fragment thereof according to any one of claims 8 to 11, wherein the antibody or the antigen-binding fragment thereof is of IgG1 (λ) class (subclass).

13. The antibody or the antigen-binding fragment thereof according to any one of claims 8 to 12, wherein the antibody or the antigen-binding fragment thereof has a neutralizing activity (EC50) of 0.1 µg/mL (approximately 0.7 nM) or lower against the *Clostridium difficile* toxin B in measurement using a human lung fibroblast IMR-90.

14. A pharmaceutical composition comprising an antibody or an antigen-binding fragment thereof according to any one of claims 1 to 13 and a pharmaceutically acceptable carrier.

15. The pharmaceutical composition according to claim 14, wherein the pharmaceutical composition comprises
(a) a first monoclonal antibody specifically binding to *Clostridium difficile* toxin A protein or an antigen-binding fragment thereof according to any one of claims 1 to 7, and
(b) a second monoclonal antibody specifically binding to *Clostridium difficile* toxin B protein or an antigen-binding fragment thereof according to any one of claims 8 to 13.

16. The pharmaceutical composition according to claim 15, wherein the first and second monoclonal antibodies or an antigen-binding fragments thereof neutralize the *Clostridium difficile* toxin A and the *Clostridium difficile* toxin B, respectively, *in vitro* or *in vivo.*

17. The pharmaceutical composition according to any one of claims 14 to 16, wherein the pharmaceutical composition is intended for the treatment of *Clostridium difficile* infection.

18. An isolated nucleic acid encoding the amino acid sequence of an antibody or an antigen-binding fragment thereof according to any one of claims 1 to 7, an isolated nucleic acid comprising a nucleotide sequence of SEQ ID NO: 1 or 7, or an isolated nucleic acid hybridizing under high stringent conditions to any of these nucleic acids.

19. A vector comprising an isolated nucleic acid according to claim 18 incorporated therein.

20. A host cell comprising a recombinant expression vector according to claim 19 introduced thereinto.

21. An isolated nucleic acid encoding the amino acid sequence of an antibody or an antigen-binding fragment thereof according to any one of claims 8 to 13, an isolated nucleic acid comprising a nucleotide sequence of SEQ ID NO: 13 or 19, or an isolated nucleic acid hybridizing under high stringent conditions to any of these nucleic acids.

22. A vector comprising an isolated nucleic acid according to claim 21 incorporated therein.

23. A host cell comprising a recombinant expression vector according to claim 22 introduced thereinto.


SEQUENCE LISTING

<110>  Evec, Inc.

<120>  Human Antibody Specific to Toxins of Clostridium Difficile and
       Its Antigen-Binding Fragment

<130>  PCT13-0038

<150>  JP 2012-232323
<151>  2012-10-19

<160>  26

<170>  PatentIn version 3.5

<210>  1
<211>  1434
<212>  DNA
<213>  Homo sapiens

<400>  1
atggactgga cctggaggtt cctctttgtg gtggcagcag ctacaggtgt ccagtcccag      60

gtccagctgg tgcagtctgg ggctgaggtg aagaagcctg ggtcctcggt gaaggtctcc     120

tgcaaggctt ctggagacac cttcagcagc catgctatca cctgggtgcg acaggcccct     180

ggacaagggc ttgagtggat gggcaggatc atccccatct ttagtatttc agactacgca     240

cagaagttcc agggcagagt cacgtttact gcggacaaat ccacgagcat agccttcatg     300

gagctgagca gcctgagatc tcaggacacg gccgtctatt actgtgcgag atcattgctg     360

gcatattgta ctggtggtag ttgctccgaa ttcggggcgg aagaccactg gggccaggga     420

accctggtca ccgtctcctc agcctccacc aagggcccat cggtcttccc cctggcaccc     480

tcctccaaga gcacctctgg gggcacagcg gccctgggct gcctggtcaa ggactacttc     540

cccgaaccgg tgacggtgtc gtggaactca ggcgccctga ccagcggcgt gcacaccttc     600

ccggctgtcc tacagtcctc aggactctac tccctcagca gcgtggtgac cgtgccctcc     660

agcagcttgg gcacccagac ctacatctgc aacgtgaatc acaagcccag caacaccaag     720

gtggacaaga aagttgagcc caaatcttgt gacaaaactc acacatgccc accgtgccca     780

gcacctgaac tcctgggggg accgtcagtc ttcctcttcc ccccaaaacc caaggacacc     840

ctcatgatct cccggacccc tgaggtcaca tgcgtggtgg tggacgtgag ccacgaagac     900

cctgaggtca agttcaactg gtacgtggac ggcgtggagg tgcataatgc caagacaaag     960

ccgcgggagg agcagtacaa cagcacgtac cgtgtggtca gcgtcctcac cgtcctgcac    1020

caggactggc tgaatggcaa ggagtacaag tgcaaggtct ccaacaaagc cctcccagcc    1080

cccatcgaga aaaccatctc caaagccaaa gggcagcccc gagaaccaca ggtgtacacc    1140

ctgcccccat cccgggatga gctgaccaag aaccaggtca gcctgacctg cctggtcaaa    1200

ggcttctatc ccagcgacat cgccgtggag tgggagagca atgggcagcc ggagaacaac    1260

tacaagacca cgcctcccgt gctggactcc gacggctcct tcttcctcta cagcaagctc 1320

accgtggaca agagcaggtg gcagcagggg aacgtcttct catgctccgt gatgcatgag 1380

gctctgcaca accactacac gcagaagagc ctctccctgt ctccgggtaa atga 1434

<210> 2
<211> 477
<212> PRT
<213> Homo sapiens

<400> 2

Met Asp Trp Thr Trp Arg Phe Leu Phe Val Val Ala Ala Ala Thr Gly
1               5                   10                  15

Val Gln Ser Gln Val Gln Leu Val Gln Ser Gly Ala Glu Val Lys Lys
            20                  25                  30

Pro Gly Ser Ser Val Lys Val Ser Cys Lys Ala Ser Gly Asp Thr Phe
        35                  40                  45

Ser Ser His Ala Ile Thr Trp Val Arg Gln Ala Pro Gly Gln Gly Leu
    50                  55                  60

Glu Trp Met Gly Arg Ile Ile Pro Ile Phe Ser Ile Ser Asp Tyr Ala
65                  70                  75                  80

Gln Lys Phe Gln Gly Arg Val Thr Phe Thr Ala Asp Lys Ser Thr Ser
                85                  90                  95

Ile Ala Phe Met Glu Leu Ser Ser Leu Arg Ser Gln Asp Thr Ala Val
            100                 105                 110

Tyr Tyr Cys Ala Arg Ser Leu Leu Ala Tyr Cys Thr Gly Gly Ser Cys
        115                 120                 125

Ser Glu Phe Gly Ala Glu Asp His Trp Gly Gln Gly Thr Leu Val Thr
    130                 135                 140

Val Ser Ser Ala Ser Thr Lys Gly Pro Ser Val Phe Pro Leu Ala Pro
145                 150                 155                 160

Ser Ser Lys Ser Thr Ser Gly Gly Thr Ala Ala Leu Gly Cys Leu Val
                165                 170                 175

Lys Asp Tyr Phe Pro Glu Pro Val Thr Val Ser Trp Asn Ser Gly Ala
            180                 185                 190

Leu Thr Ser Gly Val His Thr Phe Pro Ala Val Leu Gln Ser Ser Gly
        195                 200                 205

Leu Tyr Ser Leu Ser Ser Val Val Thr Val Pro Ser Ser Ser Leu Gly
    210                 215                 220

Thr Gln Thr Tyr Ile Cys Asn Val Asn His Lys Pro Ser Asn Thr Lys
225                 230                 235                 240

Val Asp Lys Lys Val Glu Pro Lys Ser Cys Asp Lys Thr His Thr Cys
                245                 250                 255

Pro Pro Cys Pro Ala Pro Glu Leu Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser His Glu Asp Pro Glu Val Lys
    290                 295                 300

Phe Asn Trp Tyr Val Asp Gly Val Glu Val His Asn Ala Lys Thr Lys
305                 310                 315                 320

Pro Arg Glu Glu Gln Tyr Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Ala Leu Pro Ala Pro Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Arg Asp Glu Leu Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Lys Leu Thr Val Asp Lys Ser Arg Trp Gln
        435                 440                 445

Gln Gly Asn Val Phe Ser Cys Ser Val Met His Glu Ala Leu His Asn
    450                 455                 460

His Tyr Thr Gln Lys Ser Leu Ser Leu Ser Pro Gly Lys
465                 470                 475

<210>  3
<211>  128
<212>  PRT
<213>  Homo sapiens

<400>  3

Gln Val Gln Leu Val Gln Ser Gly Ala Glu Val Lys Lys Pro Gly Ser
1               5                   10                  15

Ser Val Lys Val Ser Cys Lys Ala Ser Gly Asp Thr Phe Ser Ser His
            20                  25                  30

Ala Ile Thr Trp Val Arg Gln Ala Pro Gly Gln Gly Leu Glu Trp Met
        35                  40                  45

Gly Arg Ile Ile Pro Ile Phe Ser Ile Ser Asp Tyr Ala Gln Lys Phe
    50                  55                  60

Gln Gly Arg Val Thr Phe Thr Ala Asp Lys Ser Thr Ser Ile Ala Phe
65                  70                  75                  80

Met Glu Leu Ser Ser Leu Arg Ser Gln Asp Thr Ala Val Tyr Tyr Cys
                85                  90                  95

Ala Arg Ser Leu Leu Ala Tyr Cys Thr Gly Gly Ser Cys Ser Glu Phe
            100                 105                 110

Gly Ala Glu Asp His Trp Gly Gln Gly Thr Leu Val Thr Val Ser Ser
        115                 120                 125

<210>  4
<211>  5
<212>  PRT
<213>  Homo sapiens

<400>  4

Ser His Ala Ile Thr
1               5

<210>  5
<211>  17
<212>  PRT
<213>  Homo sapiens

<400>  5

Arg Ile Ile Pro Ile Phe Ser Ile Ser Asp Tyr Ala Gln Lys Phe Gln
1               5                   10                  15

Gly

<210>  6
<211>  19
<212>  PRT
<213>  Homo sapiens

<400>  6

Ser Leu Leu Ala Tyr Cys Thr Gly Gly Ser Cys Ser Glu Phe Gly Ala
1               5                   10                  15

Glu Asp His

<210>  7
<211>  711
<212>  DNA
<213>  Homo sapiens

<400>  7
atggaaaccc cagcgcagct tctcttcctc ctgctactct ggctcccaga taccaccgga      60

gaaattgtgt tgacgcagtc tccaggcacc ctgtctttgt ctccagggga aagagccacc     120

ctctcctgca gggccagtca gagtgttagc agcaactact tagcctggta ccagcagaaa     180

cctggccagg ctcccaggct cctcatcttt ggtgcatcca gcagggccac tggcatccca     240

gacaggttca gtggcagtgg gtctgggaca gacttcactc tcaccatcag cagactggag     300

cctgaagatt ttgcagtgta ttactgtcag cagtatggta gctcaattcc cgtgacgttc     360

ggccaaggga ccaaggtgga gatcaaacga actgtggctg caccatctgt cttcatcttc     420

ccgccatctg atgagcagtt gaaatctgga actgcctctg ttgtgtgcct gctgaataac     480

ttctatccca gagaggccaa agtacagtgg aaggtggata acgccctcca atcgggtaac     540

tcccaggaga gtgtcacaga gcaggacagc aaggacagca cctacagcct cagcagcacc     600

ctgacgctga gcaaagcaga ctacgagaaa cacaaagtct acgcctgcga agtcacccat     660

cagggcctga gctcgcccgt cacaaagagc ttcaacaggg gagagtgtta g              711

<210>  8
<211>  236
<212>  PRT
<213>  Homo sapiens

<400>  8

Met Glu Thr Pro Ala Gln Leu Leu Phe Leu Leu Leu Leu Trp Leu Pro
1               5                   10                  15

Asp Thr Thr Gly Glu Ile Val Leu Thr Gln Ser Pro Gly Thr Leu Ser
            20                  25                  30

Leu Ser Pro Gly Glu Arg Ala Thr Leu Ser Cys Arg Ala Ser Gln Ser
        35                  40                  45

Val Ser Ser Asn Tyr Leu Ala Trp Tyr Gln Gln Lys Pro Gly Gln Ala
    50                  55                  60

Pro Arg Leu Leu Ile Phe Gly Ala Ser Ser Arg Ala Thr Gly Ile Pro
65                  70                  75                  80

Asp Arg Phe Ser Gly Ser Gly Ser Gly Thr Asp Phe Thr Leu Thr Ile
                85                  90                  95

Ser Arg Leu Glu Pro Glu Asp Phe Ala Val Tyr Tyr Cys Gln Gln Tyr
            100                 105                 110

Gly Ser Ser Ile Pro Val Thr Phe Gly Gln Gly Thr Lys Val Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235

<210>  9
<211>  109

<212>  PRT
<213>  Homo sapiens

<400>  9

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Asn
            20                  25                  30

Tyr Leu Ala Trp Tyr Gln Gln Lys Pro Gly Gln Ala Pro Arg Leu Leu
        35                  40                  45

Ile Phe Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Ile
                85                  90                  95

Pro Val Thr Phe Gly Gln Gly Thr Lys Val Glu Ile Lys
            100                 105

<210>  10
<211>  12
<212>  PRT
<213>  Homo sapiens

<400>  10

Arg Ala Ser Gln Ser Val Ser Ser Asn Tyr Leu Ala
1               5                   10

<210>  11
<211>  7
<212>  PRT
<213>  Homo sapiens

<400>  11

Gly Ala Ser Ser Arg Ala Thr
1               5

<210>  12
<211>  10
<212>  PRT
<213>  Homo sapiens

<400>  12

Gln Gln Tyr Gly Ser Ser Ile Pro Val Thr

1               5                   10

<210>  13
<211>  1410
<212>  DNA
<213>  Homo sapiens

<400>  13
atggaattgg ggctgagctg ggttttcctt gttgctcttt tagaaggtgt ccactgtgag      60

gtgcaactgg tggagtctgg gggaggcttg gtccagccgg gggggtccct cagactctcc     120

tgtgcagcct ctggactcac cattaatagc tattggatga actgggtccg ccaaattcca     180

gggaaggggc tggagtgggt ggccaacata aacccaactg gaagtcagca atattatgtg     240

gactctgtga ggggccgatt caccatctcc agagacgacg ccaagaagtc actgtttctg     300

caattggaca gcctcagagt cgaggacacg gctgtgtatt actgtgcgag aggccgtcgg     360

ggtaactccg cctactgtga ttcctggggc cagggaaccc aggtcaccgt ctcctcagcc     420

tccaccaagg gcccatcggt cttccccctg gcaccctcct ccaagagcac ctctgggggc     480

acagcggccc tgggctgcct ggtcaaggac tacttccccg aaccggtgac ggtgtcgtgg     540

aactcaggcg ccctgaccag cggcgtgcac accttcccgg ctgtcctaca gtcctcagga     600

ctctactccc tcagcagcgt ggtgaccgtg ccctccagca gcttgggcac ccagacctac     660

atctgcaacg tgaatcacaa gcccagcaac accaaggtgg acaagaaagt tgagcccaaa     720

tcttgtgaca aaactcacac atgcccaccg tgcccagcac ctgaactcct ggggggaccg     780

tcagtcttcc tcttcccccc aaaacccaag gacaccctca tgatctcccg gacccctgag     840

gtcacatgcg tggtggtgga cgtgagccac gaagaccctg aggtcaagtt caactggtac     900

gtggacggcg tggaggtgca taatgccaag acaaagccgc gggaggagca gtacaacagc     960

acgtaccgtg tggtcagcgt cctcaccgtc ctgcaccagg actggctgaa tggcaaggag    1020

tacaagtgca aggtctccaa caaagccctc ccagccccca tcgagaaaac catctccaaa    1080

gccaaagggc agccccgaga accacaggtg tacaccctgc ccccatcccg ggatgagctg    1140

accaagaacc aggtcagcct gacctgcctg gtcaaaggct tctatcccag cgacatcgcc    1200

gtggagtggg agagcaatgg gcagccggag aacaactaca agaccacgcc tcccgtgctg    1260

gactccgacg gctccttctt cctctacagc aagctcaccg tggacaagag caggtggcag    1320

caggggaacg tcttctcatg ctccgtgatg catgaggctc tgcacaacca ctacacgcag    1380

aagagcctct ccctgtctcc gggtaaatga                                    1410

<210>  14
<211>  469
<212>  PRT
<213>  Homo sapiens

<400>  14

<210> 15
<211> 120
<212> PRT
<213> Homo sapiens

<400>  15

Glu Val Gln Leu Val Glu Ser Gly Gly Gly Leu Val Gln Pro Gly Gly
1               5                   10                  15

Ser Leu Arg Leu Ser Cys Ala Ala Ser Gly Leu Thr Ile Asn Ser Tyr
            20                  25                  30

Trp Met Asn Trp Val Arg Gln Ile Pro Gly Lys Gly Leu Glu Trp Val
        35                  40                  45

Ala Asn Ile Asn Pro Thr Gly Ser Gln Gln Tyr Tyr Val Asp Ser Val
    50                  55                  60

Arg Gly Arg Phe Thr Ile Ser Arg Asp Asp Ala Lys Lys Ser Leu Phe
65                  70                  75                  80

Leu Gln Leu Asp Ser Leu Arg Val Glu Asp Thr Ala Val Tyr Tyr Cys
                85                  90                  95

Ala Arg Gly Arg Arg Gly Asn Ser Ala Tyr Cys Asp Ser Trp Gly Gln
            100                 105                 110

Gly Thr Gln Val Thr Val Ser Ser
        115                 120


<210>  16
<211>  5
<212>  PRT
<213>  Homo sapiens

<400>  16

Ser Tyr Trp Met Asn
1               5


<210>  17
<211>  17
<212>  PRT
<213>  Homo sapiens

<400>  17

Asn Ile Asn Pro Thr Gly Ser Gln Gln Tyr Tyr Val Asp Ser Val Arg
1               5                   10                  15

Gly


<210>  18
<211>  11

<212>  PRT
<213>  Homo sapiens

<400>  18

Gly Arg Arg Gly Asn Ser Ala Tyr Cys Asp Ser
1               5                   10


<210>  19
<211>  708
<212>  DNA
<213>  Homo sapiens

<400>  19
atggcctggg ctctgctcct cctcagcctc ctcactcagg gcacaggatc ctgggctcag        60

tctgccctga ctcagcctcg ctcagtgtcc ggttcccctg gacagtcagt caccatctcc       120

tgcactggaa ccagtagtga tattggtgca tatgattatg tctcgtggta tcgacaccac       180

cccggcaaag cccccaaact catcattttt gatgtcgcca agtggccctc aggggtccca       240

gatcgcttct ctggctccaa gtctggcaac acggcctccc tgaccatctc tgggctccag       300

gatgacgatg agggcgatta ttactgctgc tcatatgcgg gaagattcag tctaatattc       360

ggcgggggga ccaagttgac cgtcctaggt cagcccaagg ctgccccctc ggtcactctg       420

ttcccgccct cctctgagga gcttcaagcc aacaaggcca cactggtgtg tctcataagt       480

gacttctacc cgggagccgt gacagtggcc tggaaggcag atagcagccc cgtcaaggcg       540

ggagtggaga ccaccacacc ctccaaacaa agcaacaaca agtacgcggc cagcagctac       600

ctgagcctga cgcctgagca gtggaagtcc cacagaagct acagctgcca ggtcacgcat       660

gaagggagca ccgtggagaa gacagtggcc cctacagaat gttcatag                    708


<210>  20
<211>  235
<212>  PRT
<213>  Homo sapiens

<400>  20

Met Ala Trp Ala Leu Leu Leu Leu Ser Leu Leu Thr Gln Gly Thr Gly
1               5                   10                  15


Ser Trp Ala Gln Ser Ala Leu Thr Gln Pro Arg Ser Val Ser Gly Ser
            20                  25                  30


Pro Gly Gln Ser Val Thr Ile Ser Cys Thr Gly Thr Ser Ser Asp Ile
        35                  40                  45


Gly Ala Tyr Asp Tyr Val Ser Trp Tyr Arg His His Pro Gly Lys Ala
    50                  55                  60

Pro Lys Leu Ile Ile Phe Asp Val Ala Lys Trp Pro Ser Gly Val Pro
65                  70                  75                  80

Asp Arg Phe Ser Gly Ser Lys Ser Gly Asn Thr Ala Ser Leu Thr Ile
                85                  90                  95

Ser Gly Leu Gln Asp Asp Asp Glu Gly Asp Tyr Tyr Cys Cys Ser Tyr
            100                 105                 110

Ala Gly Arg Phe Ser Leu Ile Phe Gly Gly Gly Thr Lys Leu Thr Val
        115                 120                 125

Leu Gly Gln Pro Lys Ala Ala Pro Ser Val Thr Leu Phe Pro Pro Ser
    130                 135                 140

Ser Glu Glu Leu Gln Ala Asn Lys Ala Thr Leu Val Cys Leu Ile Ser
145                 150                 155                 160

Asp Phe Tyr Pro Gly Ala Val Thr Val Ala Trp Lys Ala Asp Ser Ser
                165                 170                 175

Pro Val Lys Ala Gly Val Glu Thr Thr Thr Pro Ser Lys Gln Ser Asn
            180                 185                 190

Asn Lys Tyr Ala Ala Ser Ser Tyr Leu Ser Leu Thr Pro Glu Gln Trp
        195                 200                 205

Lys Ser His Arg Ser Tyr Ser Cys Gln Val Thr His Glu Gly Ser Thr
    210                 215                 220

Val Glu Lys Thr Val Ala Pro Thr Glu Cys Ser
225                 230                 235

<210>  21
<211>  111
<212>  PRT
<213>  Homo sapiens

<400>  21

Gln Ser Ala Leu Thr Gln Pro Arg Ser Val Ser Gly Ser Pro Gly Gln
1               5                   10                  15

Ser Val Thr Ile Ser Cys Thr Gly Thr Ser Ser Asp Ile Gly Ala Tyr
            20                  25                  30

Asp Tyr Val Ser Trp Tyr Arg His His Pro Gly Lys Ala Pro Lys Leu
        35                  40                  45

Ile Ile Phe Asp Val Ala Lys Trp Pro Ser Gly Val Pro Asp Arg Phe
    50                  55                  60

Ser Gly Ser Lys Ser Gly Asn Thr Ala Ser Leu Thr Ile Ser Gly Leu
65                  70                  75                  80

Gln Asp Asp Asp Glu Gly Asp Tyr Tyr Cys Cys Ser Tyr Ala Gly Arg
                85                  90                  95

Phe Ser Leu Ile Phe Gly Gly Gly Thr Lys Leu Thr Val Leu Gly
            100                 105                 110

<210>  22
<211>  14
<212>  PRT
<213>  Homo sapiens

<400>  22

Thr Gly Thr Ser Ser Asp Ile Gly Ala Tyr Asp Tyr Val Ser
1               5                   10

<210>  23
<211>  7
<212>  PRT
<213>  Homo sapiens

<400>  23

Asp Val Ala Lys Trp Pro Ser
1               5

<210>  24
<211>  10
<212>  PRT
<213>  Homo sapiens

<400>  24

Cys Ser Tyr Ala Gly Arg Phe Ser Leu Ile
1               5                   10

<210>  25
<211>  2710
<212>  PRT
<213>  Homo sapiens

<400>  25

Met Ser Leu Ile Ser Lys Glu Glu Leu Ile Lys Leu Ala Tyr Ser Ile
1               5                   10                  15

Arg Pro Arg Glu Asn Glu Tyr Lys Thr Ile Leu Thr Asn Leu Asp Glu
            20                  25                  30

Tyr Asn Lys Leu Thr Thr Asn Asn Asn Glu Asn Lys Tyr Leu Gln Leu
        35                  40                  45

Lys Lys Leu Asn Glu Ser Ile Asp Val Phe Met Asn Lys Tyr Lys Thr
    50                  55                  60

Ser Ser Arg Asn Arg Ala Leu Ser Asn Leu Lys Lys Asp Ile Leu Lys
65                  70                  75                  80

Glu Val Ile Leu Ile Lys Asn Ser Asn Thr Ser Pro Val Glu Lys Asn
                85                  90                  95

Leu His Phe Val Trp Ile Gly Gly Glu Val Ser Asp Ile Ala Leu Glu
            100                 105                 110

Tyr Ile Lys Gln Trp Ala Asp Ile Asn Ala Glu Tyr Asn Ile Lys Leu
        115                 120                 125

Trp Tyr Asp Ser Glu Ala Phe Leu Val Asn Thr Leu Lys Lys Ala Ile
    130                 135                 140

Val Glu Ser Ser Thr Thr Glu Ala Leu Gln Leu Leu Glu Glu Glu Ile
145                 150                 155                 160

Gln Asn Pro Gln Phe Asp Asn Met Lys Phe Tyr Lys Lys Arg Met Glu
                165                 170                 175

Phe Ile Tyr Asp Arg Gln Lys Arg Phe Ile Asn Tyr Tyr Lys Ser Gln
            180                 185                 190

Ile Asn Lys Pro Thr Val Pro Thr Ile Asp Asp Ile Ile Lys Ser His
        195                 200                 205

Leu Val Ser Glu Tyr Asn Arg Asp Glu Thr Val Leu Glu Ser Tyr Arg
    210                 215                 220

Thr Asn Ser Leu Arg Lys Ile Asn Ser Asn His Gly Ile Asp Ile Arg
225                 230                 235                 240

Ala Asn Ser Leu Phe Thr Glu Gln Glu Leu Leu Asn Ile Tyr Ser Gln
                245                 250                 255

Glu Leu Leu Asn Arg Gly Asn Leu Ala Ala Ala Ser Asp Ile Val Arg
            260                 265                 270

Leu Leu Ala Leu Lys Asn Phe Gly Gly Val Tyr Leu Asp Val Asp Met
        275                 280                 285

Leu Pro Gly Ile His Ser Asp Leu Phe Lys Thr Ile Ser Arg Pro Ser
    290                 295                 300

Ser Ile Gly Leu Asp Arg Trp Glu Met Ile Lys Leu Glu Ala Ile Met
305                 310                 315                 320

Lys Tyr Lys Lys Tyr Ile Asn Asn Tyr Thr Ser Glu Asn Phe Asp Lys
                325                 330                 335

Leu Asp Gln Gln Leu Lys Asp Asn Phe Lys Leu Ile Ile Glu Ser Lys
            340                 345                 350

Ser Glu Lys Ser Glu Ile Phe Ser Lys Leu Glu Asn Leu Asn Val Ser
        355                 360                 365

Asp Leu Glu Ile Lys Ile Ala Phe Ala Leu Gly Ser Val Ile Asn Gln
    370                 375                 380

Ala Leu Ile Ser Lys Gln Gly Ser Tyr Leu Thr Asn Leu Val Ile Glu
385                 390                 395                 400

Gln Val Lys Asn Arg Tyr Gln Phe Leu Asn Gln His Leu Asn Pro Ala
                405                 410                 415

Ile Glu Ser Asp Asn Asn Phe Thr Asp Thr Thr Lys Ile Phe His Asp
            420                 425                 430

Ser Leu Phe Asn Ser Ala Thr Ala Glu Asn Ser Met Phe Leu Thr Lys
        435                 440                 445

Ile Ala Pro Tyr Leu Gln Val Gly Phe Met Pro Glu Ala Arg Ser Thr
    450                 455                 460

Ile Ser Leu Ser Gly Pro Gly Ala Tyr Ala Ser Ala Tyr Tyr Asp Phe
465                 470                 475                 480

Ile Asn Leu Gln Glu Asn Thr Ile Glu Lys Thr Leu Lys Ala Ser Asp
                485                 490                 495

Leu Ile Glu Phe Lys Phe Pro Glu Asn Asn Leu Ser Gln Leu Thr Glu
            500                 505                 510

Gln Glu Ile Asn Ser Leu Trp Ser Phe Asp Gln Ala Ser Ala Lys Tyr
        515                 520                 525

Gln Phe Glu Lys Tyr Val Arg Asp Tyr Thr Gly Gly Ser Leu Ser Glu

530                 535                 540

Asp Asn Gly Val Asp Phe Asn Lys Asn Thr Ala Leu Asp Lys Asn Tyr
545                 550                 555                 560

Leu Leu Asn Asn Lys Ile Pro Ser Asn Asn Val Glu Glu Ala Gly Ser
                565                 570                 575

Lys Asn Tyr Val His Tyr Ile Ile Gln Leu Gln Gly Asp Asp Ile Ser
            580                 585                 590

Tyr Glu Ala Thr Cys Asn Leu Phe Ser Lys Asn Pro Lys Asn Ser Ile
        595                 600                 605

Ile Ile Gln Arg Asn Met Asn Glu Ser Ala Lys Ser Tyr Phe Leu Ser
    610                 615                 620

Asp Asp Gly Glu Ser Ile Leu Glu Leu Asn Lys Tyr Arg Ile Pro Glu
625                 630                 635                 640

Arg Leu Lys Asn Lys Glu Lys Val Lys Val Thr Phe Ile Gly His Gly
                645                 650                 655

Lys Asp Glu Phe Asn Thr Ser Glu Phe Ala Arg Leu Ser Val Asp Ser
            660                 665                 670

Leu Ser Asn Glu Ile Ser Ser Phe Leu Asp Thr Ile Lys Leu Asp Ile
        675                 680                 685

Ser Pro Lys Asn Val Glu Val Asn Leu Leu Gly Cys Asn Met Phe Ser
    690                 695                 700

Tyr Asp Phe Asn Val Glu Glu Thr Tyr Pro Gly Lys Leu Leu Leu Ser
705                 710                 715                 720

Ile Met Asp Lys Ile Thr Ser Thr Leu Pro Asp Val Asn Lys Asn Ser
                725                 730                 735

Ile Thr Ile Gly Ala Asn Gln Tyr Glu Val Arg Ile Asn Ser Glu Gly
            740                 745                 750

Arg Lys Glu Leu Leu Ala His Ser Gly Lys Trp Ile Asn Lys Glu Glu
        755                 760                 765

Ala Ile Met Ser Asp Leu Ser Ser Lys Glu Tyr Ile Phe Phe Asp Ser
    770                 775                 780

Ile Asp Asn Lys Leu Lys Ala Lys Ser Lys Asn Ile Pro Gly Leu Ala
785                 790                 795                 800

Ser Ile Ser Glu Asp Ile Lys Thr Leu Leu Leu Asp Ala Ser Val Ser
                805                 810                 815

Pro Asp Thr Lys Phe Ile Leu Asn Asn Leu Lys Leu Asn Ile Glu Ser
            820                 825                 830

Ser Ile Gly Asp Tyr Ile Tyr Tyr Glu Lys Leu Glu Pro Val Lys Asn
        835                 840                 845

Ile Ile His Asn Ser Ile Asp Asp Leu Ile Asp Glu Phe Asn Leu Leu
    850                 855                 860

Glu Asn Val Ser Asp Glu Leu Tyr Glu Leu Lys Lys Leu Asn Asn Leu
865                 870                 875                 880

Asp Glu Lys Tyr Leu Ile Ser Phe Glu Asp Ile Ser Lys Asn Asn Ser
                885                 890                 895

Thr Tyr Ser Val Arg Phe Ile Asn Lys Ser Asn Gly Glu Ser Val Tyr
            900                 905                 910

Val Glu Thr Glu Lys Glu Ile Phe Ser Lys Tyr Ser Glu His Ile Thr
        915                 920                 925

Lys Glu Ile Ser Thr Ile Lys Asn Ser Ile Ile Thr Asp Val Asn Gly
    930                 935                 940

Asn Leu Leu Asp Asn Ile Gln Leu Asp His Thr Ser Gln Val Asn Thr
945                 950                 955                 960

Leu Asn Ala Ala Phe Phe Ile Gln Ser Leu Ile Asp Tyr Ser Ser Asn
                965                 970                 975

Lys Asp Val Leu Asn Asp Leu Ser Thr Ser Val Lys Val Gln Leu Tyr
            980                 985                 990

Ala Gln Leu Phe Ser Thr Gly Leu  Asn Thr Ile Tyr Asp  Ser Ile Gln
        995                 1000                 1005

Leu Val  Asn Leu Ile Ser Asn  Ala Val Asn Asp Thr  Ile Asn Val
    1010                 1015                 1020

Leu Pro  Thr Ile Thr Glu Gly  Ile Pro Ile Val Ser  Thr Ile Leu
    1025                 1030                 1035

Asp Gly Ile Asn Leu Gly Ala Ala Ile Lys Glu Leu Leu Asp Glu
    1040                1045                1050

His Asp Pro Leu Leu Lys Lys Glu Leu Glu Ala Lys Val Gly Val
    1055                1060                1065

Leu Ala Ile Asn Met Ser Leu Ser Ile Ala Ala Thr Val Ala Ser
    1070                1075                1080

Ile Val Gly Ile Gly Ala Glu Val Thr Ile Phe Leu Leu Pro Ile
    1085                1090                1095

Ala Gly Ile Ser Ala Gly Ile Pro Ser Leu Val Asn Asn Glu Leu
    1100                1105                1110

Ile Leu His Asp Lys Ala Thr Ser Val Val Asn Tyr Phe Asn His
    1115                1120                1125

Leu Ser Glu Ser Lys Lys Tyr Gly Pro Leu Lys Thr Glu Asp Asp
    1130                1135                1140

Lys Ile Leu Val Pro Ile Asp Asp Leu Val Ile Ser Glu Ile Asp
    1145                1150                1155

Phe Asn Asn Asn Ser Ile Lys Leu Gly Thr Cys Asn Ile Leu Ala
    1160                1165                1170

Met Glu Gly Gly Ser Gly His Thr Val Thr Gly Asn Ile Asp His
    1175                1180                1185

Phe Phe Ser Ser Pro Ser Ile Ser Ser His Ile Pro Ser Leu Ser
    1190                1195                1200

Ile Tyr Ser Ala Ile Gly Ile Glu Thr Glu Asn Leu Asp Phe Ser
    1205                1210                1215

Lys Lys Ile Met Met Leu Pro Asn Ala Pro Ser Arg Val Phe Trp
    1220                1225                1230

Trp Glu Thr Gly Ala Val Pro Gly Leu Arg Ser Leu Glu Asn Asp
    1235                1240                1245

Gly Thr Arg Leu Leu Asp Ser Ile Arg Asp Leu Tyr Pro Gly Lys
    1250                1255                1260

Phe Tyr Trp Arg Phe Tyr Ala Phe Phe Asp Tyr Ala Ile Thr Thr
    1265                1270                1275

Leu Lys Pro Val Tyr Glu Asp Thr Asn Ile Lys Ile Lys Leu Asp
    1280                1285                1290

Lys Asp Thr Arg Asn Phe Ile Met Pro Thr Ile Thr Thr Asn Glu
    1295                1300                1305

Ile Arg Asn Lys Leu Ser Tyr Ser Phe Asp Gly Ala Gly Gly Thr
    1310                1315                1320

Tyr Ser Leu Leu Leu Ser Ser Tyr Pro Ile Ser Thr Asn Ile Asn
    1325                1330                1335

Leu Ser Lys Asp Asp Leu Trp Ile Phe Asn Ile Asp Asn Glu Val
    1340                1345                1350

Arg Glu Ile Ser Ile Glu Asn Gly Thr Ile Lys Lys Gly Lys Leu
    1355                1360                1365

Ile Lys Asp Val Leu Ser Lys Ile Asp Ile Asn Lys Asn Lys Leu
    1370                1375                1380

Ile Ile Gly Asn Gln Thr Ile Asp Phe Ser Gly Asp Ile Asp Asn
    1385                1390                1395

Lys Asp Arg Tyr Ile Phe Leu Thr Cys Glu Leu Asp Asp Lys Ile
    1400                1405                1410

Ser Leu Ile Ile Glu Ile Asn Leu Val Ala Lys Ser Tyr Ser Leu
    1415                1420                1425

Leu Leu Ser Gly Asp Lys Asn Tyr Leu Ile Ser Asn Leu Ser Asn
    1430                1435                1440

Thr Ile Glu Lys Ile Asn Thr Leu Gly Leu Asp Ser Lys Asn Ile
    1445                1450                1455

Ala Tyr Asn Tyr Thr Asp Glu Ser Asn Asn Lys Tyr Phe Gly Ala
    1460                1465                1470

Ile Ser Lys Thr Ser Gln Lys Ser Ile Ile His Tyr Lys Lys Asp
    1475                1480                1485

Ser Lys Asn Ile Leu Glu Phe Tyr Asn Asp Ser Thr Leu Glu Phe
    1490                1495                1500

Asn Ser Lys Asp Phe Ile Ala Glu Asp Ile Asn Val Phe Met Lys

1505                1510                1515

Asp Asp Ile Asn Thr Ile Thr Gly Lys Tyr Tyr Val Asp Asn Asn
    1520                1525                1530

Thr Asp Lys Ser Ile Asp Phe Ser Ile Ser Leu Val Ser Lys Asn
    1535                1540                1545

Gln Val Lys Val Asn Gly Leu Tyr Leu Asn Glu Ser Val Tyr Ser
    1550                1555                1560

Ser Tyr Leu Asp Phe Val Lys Asn Ser Asp Gly His His Asn Thr
    1565                1570                1575

Ser Asn Phe Met Asn Leu Phe Leu Asp Asn Ile Ser Phe Trp Lys
    1580                1585                1590

Leu Phe Gly Phe Glu Asn Ile Asn Phe Val Ile Asp Lys Tyr Phe
    1595                1600                1605

Thr Leu Val Gly Lys Thr Asn Leu Gly Tyr Val Glu Phe Ile Cys
    1610                1615                1620

Asp Asn Asn Lys Asn Ile Asp Ile Tyr Phe Gly Glu Trp Lys Thr
    1625                1630                1635

Ser Ser Ser Lys Ser Thr Ile Phe Ser Gly Asn Gly Arg Asn Val
    1640                1645                1650

Val Val Glu Pro Ile Tyr Asn Pro Asp Thr Gly Glu Asp Ile Ser
    1655                1660                1665

Thr Ser Leu Asp Phe Ser Tyr Glu Pro Leu Tyr Gly Ile Asp Arg
    1670                1675                1680

Tyr Ile Asn Lys Val Leu Ile Ala Pro Asp Leu Tyr Thr Ser Leu
    1685                1690                1695

Ile Asn Ile Asn Thr Asn Tyr Tyr Ser Asn Glu Tyr Tyr Pro Glu
    1700                1705                1710

Ile Ile Val Leu Asn Pro Asn Thr Phe His Lys Lys Val Asn Ile
    1715                1720                1725

Asn Leu Asp Ser Ser Ser Phe Glu Tyr Lys Trp Ser Thr Glu Gly
    1730                1735                1740

Ser Asp Phe Ile Leu Val Arg Tyr Leu Glu Glu Ser Asn Lys Lys
    1745                1750                1755

Ile Leu Gln Lys Ile Arg Ile Lys Gly Ile Leu Ser Asn Thr Gln
    1760                1765                1770

Ser Phe Asn Lys Met Ser Ile Asp Phe Lys Asp Ile Lys Lys Leu
    1775                1780                1785

Ser Leu Gly Tyr Ile Met Ser Asn Phe Lys Ser Phe Asn Ser Glu
    1790                1795                1800

Asn Glu Leu Asp Arg Asp His Leu Gly Phe Lys Ile Ile Asp Asn
    1805                1810                1815

Lys Thr Tyr Tyr Tyr Asp Glu Asp Ser Lys Leu Val Lys Gly Leu
    1820                1825                1830

Ile Asn Ile Asn Asn Ser Leu Phe Tyr Phe Asp Pro Ile Glu Phe
    1835                1840                1845

Asn Leu Val Thr Gly Trp Gln Thr Ile Asn Gly Lys Lys Tyr Tyr
    1850                1855                1860

Phe Asp Ile Asn Thr Gly Ala Ala Leu Thr Ser Tyr Lys Ile Ile
    1865                1870                1875

Asn Gly Lys His Phe Tyr Phe Asn Asn Asp Gly Val Met Gln Leu
    1880                1885                1890

Gly Val Phe Lys Gly Pro Asp Gly Phe Glu Tyr Phe Ala Pro Ala
    1895                1900                1905

Asn Thr Gln Asn Asn Asn Ile Glu Gly Gln Ala Ile Val Tyr Gln
    1910                1915                1920

Ser Lys Phe Leu Thr Leu Asn Gly Lys Lys Tyr Tyr Phe Asp Asn
    1925                1930                1935

Asn Ser Lys Ala Val Thr Gly Trp Arg Ile Ile Asn Asn Glu Lys
    1940                1945                1950

Tyr Tyr Phe Asn Pro Asn Asn Ala Ile Ala Ala Val Gly Leu Gln
    1955                1960                1965

Val Ile Asp Asn Asn Lys Tyr Tyr Phe Asn Pro Asp Thr Ala Ile
    1970                1975                1980

Ile Ser Lys Gly Trp Gln Thr Val Asn Gly Ser Arg Tyr Tyr Phe
    1985                1990                1995

Asp Thr Asp Thr Ala Ile Ala Phe Asn Gly Tyr Lys Thr Ile Asp
    2000                2005                2010

Gly Lys His Phe Tyr Phe Asp Ser Asp Cys Val Val Lys Ile Gly
    2015                2020                2025

Val Phe Ser Thr Ser Asn Gly Phe Glu Tyr Phe Ala Pro Ala Asn
    2030                2035                2040

Thr Tyr Asn Asn Asn Ile Glu Gly Gln Ala Ile Val Tyr Gln Ser
    2045                2050                2055

Lys Phe Leu Thr Leu Asn Gly Lys Lys Tyr Tyr Phe Asp Asn Asn
    2060                2065                2070

Ser Lys Ala Val Thr Gly Leu Gln Thr Ile Asp Ser Lys Lys Tyr
    2075                2080                2085

Tyr Phe Asn Thr Asn Thr Ala Glu Ala Ala Thr Gly Trp Gln Thr
    2090                2095                2100

Ile Asp Gly Lys Lys Tyr Tyr Phe Asn Thr Asn Thr Ala Glu Ala
    2105                2110                2115

Ala Thr Gly Trp Gln Thr Ile Asp Gly Lys Lys Tyr Tyr Phe Asn
    2120                2125                2130

Thr Asn Thr Ala Ile Ala Ser Thr Gly Tyr Thr Ile Ile Asn Gly
    2135                2140                2145

Lys His Phe Tyr Phe Asn Thr Asp Gly Ile Met Gln Ile Gly Val
    2150                2155                2160

Phe Lys Gly Pro Asn Gly Phe Glu Tyr Phe Ala Pro Ala Asn Thr
    2165                2170                2175

Asp Ala Asn Asn Ile Glu Gly Gln Ala Ile Leu Tyr Gln Asn Glu
    2180                2185                2190

Phe Leu Thr Leu Asn Gly Lys Lys Tyr Tyr Phe Gly Ser Asp Ser
    2195                2200                2205

Lys Ala Val Thr Gly Trp Arg Ile Ile Asn Asn Lys Lys Tyr Tyr
    2210                2215                2220

Phe Asn Pro Asn Asn Ala Ile Ala Ala Ile His Leu Cys Thr Ile
    2225                2230                2235

Asn Asn Asp Lys Tyr Tyr Phe Ser Tyr Asp Gly Ile Leu Gln Asn
    2240                2245                2250

Gly Tyr Ile Thr Ile Glu Arg Asn Asn Phe Tyr Phe Asp Ala Asn
    2255                2260                2265

Asn Glu Ser Lys Met Val Thr Gly Val Phe Lys Gly Pro Asn Gly
    2270                2275                2280

Phe Glu Tyr Phe Ala Pro Ala Asn Thr His Asn Asn Asn Ile Glu
    2285                2290                2295

Gly Gln Ala Ile Val Tyr Gln Asn Lys Phe Leu Thr Leu Asn Gly
    2300                2305                2310

Lys Lys Tyr Tyr Phe Asp Asn Asp Ser Lys Ala Val Thr Gly Trp
    2315                2320                2325

Gln Thr Ile Asp Gly Lys Lys Tyr Tyr Phe Asn Leu Asn Thr Ala
    2330                2335                2340

Glu Ala Ala Thr Gly Trp Gln Thr Ile Asp Gly Lys Lys Tyr Tyr
    2345                2350                2355

Phe Asn Leu Asn Thr Ala Glu Ala Ala Thr Gly Trp Gln Thr Ile
    2360                2365                2370

Asp Gly Lys Lys Tyr Tyr Phe Asn Thr Asn Thr Phe Ile Ala Ser
    2375                2380                2385

Thr Gly Tyr Thr Ser Ile Asn Gly Lys His Phe Tyr Phe Asn Thr
    2390                2395                2400

Asp Gly Ile Met Gln Ile Gly Val Phe Lys Gly Pro Asn Gly Phe
    2405                2410                2415

Glu Tyr Phe Ala Pro Ala Asn Thr Asp Ala Asn Asn Ile Glu Gly
    2420                2425                2430

Gln Ala Ile Leu Tyr Gln Asn Lys Phe Leu Thr Leu Asn Gly Lys
    2435                2440                2445

Lys Tyr Tyr Phe Gly Ser Asp Ser Lys Ala Val Thr Gly Leu Arg

2450                2455                2460

Thr Ile Asp Gly Lys Lys Tyr Tyr Phe Asn Thr Asn Thr Ala Val
    2465                2470                2475

Ala Val Thr Gly Trp Gln Thr Ile Asn Gly Lys Lys Tyr Tyr Phe
    2480                2485                2490

Asn Thr Asn Thr Ser Ile Ala Ser Thr Gly Tyr Thr Ile Ile Ser
    2495                2500                2505

Gly Lys His Phe Tyr Phe Asn Thr Asp Gly Ile Met Gln Ile Gly
    2510                2515                2520

Val Phe Lys Gly Pro Asp Gly Phe Glu Tyr Phe Ala Pro Ala Asn
    2525                2530                2535

Thr Asp Ala Asn Asn Ile Glu Gly Gln Ala Ile Arg Tyr Gln Asn
    2540                2545                2550

Arg Phe Leu Tyr Leu His Asp Asn Ile Tyr Tyr Phe Gly Asn Asn
    2555                2560                2565

Ser Lys Ala Ala Thr Gly Trp Val Thr Ile Asp Gly Asn Arg Tyr
    2570                2575                2580

Tyr Phe Glu Pro Asn Thr Ala Met Gly Ala Asn Gly Tyr Lys Thr
    2585                2590                2595

Ile Asp Asn Lys Asn Phe Tyr Phe Arg Asn Gly Leu Pro Gln Ile
    2600                2605                2610

Gly Val Phe Lys Gly Ser Asn Gly Phe Glu Tyr Phe Ala Pro Ala
    2615                2620                2625

Asn Thr Asp Ala Asn Asn Ile Glu Gly Gln Ala Ile Arg Tyr Gln
    2630                2635                2640

Asn Arg Phe Leu His Leu Leu Gly Lys Ile Tyr Tyr Phe Gly Asn
    2645                2650                2655

Asn Ser Lys Ala Val Thr Gly Trp Gln Thr Ile Asn Gly Lys Val
    2660                2665                2670

Tyr Tyr Phe Met Pro Asp Thr Ala Met Ala Ala Ala Gly Gly Leu
    2675                2680                2685

Phe Glu Ile Asp Gly Val Ile Tyr Phe Phe Gly Val Asp Gly Val
    2690                2695                2700

Lys Ala Pro Gly Ile Tyr Gly
    2705                2710

<210> 26
<211> 2366
<212> PRT
<213> Homo sapiens

<400> 26

Met Ser Leu Val Asn Arg Lys Gln Leu Glu Lys Met Ala Asn Val Arg
1               5                   10                  15

Phe Arg Thr Gln Glu Asp Glu Tyr Val Ala Ile Leu Asp Ala Leu Glu
            20                  25                  30

Glu Tyr His Asn Met Ser Glu Asn Thr Val Val Glu Lys Tyr Leu Lys
        35                  40                  45

Leu Lys Asp Ile Asn Ser Leu Thr Asp Ile Tyr Ile Asp Thr Tyr Lys
    50                  55                  60

Lys Ser Gly Arg Asn Lys Ala Leu Lys Lys Phe Lys Glu Tyr Leu Val
65                  70                  75                  80

Thr Glu Val Leu Glu Leu Lys Asn Asn Asn Leu Thr Pro Val Glu Lys
                85                  90                  95

Asn Leu His Phe Val Trp Ile Gly Gly Gln Ile Asn Asp Thr Ala Ile
            100                 105                 110

Asn Tyr Ile Asn Gln Trp Lys Asp Val Asn Ser Asp Tyr Asn Val Asn
        115                 120                 125

Val Phe Tyr Asp Ser Asn Ala Phe Leu Ile Asn Thr Leu Lys Lys Thr
    130                 135                 140

Val Val Glu Ser Ala Ile Asn Asp Thr Leu Glu Ser Phe Arg Glu Asn
145                 150                 155                 160

Leu Asn Asp Pro Arg Phe Asp Tyr Asn Lys Phe Phe Arg Lys Arg Met
                165                 170                 175

Glu Ile Ile Tyr Asp Lys Gln Lys Asn Phe Ile Asn Tyr Tyr Lys Ala
            180                 185                 190

Gln Arg Glu Glu Asn Pro Glu Leu Ile Ile Asp Asp Ile Val Lys Thr
        195                 200                 205

Tyr Leu Ser Asn Glu Tyr Ser Lys Glu Ile Asp Glu Leu Asn Thr Tyr
    210                 215                 220

Ile Glu Glu Ser Leu Asn Lys Ile Thr Gln Asn Ser Gly Asn Asp Val
225                 230                 235                 240

Arg Asn Phe Glu Glu Phe Lys Asn Gly Glu Ser Phe Asn Leu Tyr Glu
                245                 250                 255

Gln Glu Leu Val Glu Arg Trp Asn Leu Ala Ala Ala Ser Asp Ile Leu
            260                 265                 270

Arg Ile Ser Ala Leu Lys Glu Ile Gly Gly Met Tyr Leu Asp Val Asp
        275                 280                 285

Met Leu Pro Gly Ile Gln Pro Asp Leu Phe Glu Ser Ile Glu Lys Pro
    290                 295                 300

Ser Ser Val Thr Val Asp Phe Trp Glu Met Thr Lys Leu Glu Ala Ile
305                 310                 315                 320

Met Lys Tyr Lys Glu Tyr Ile Pro Glu Tyr Thr Ser Glu His Phe Asp
                325                 330                 335

Met Leu Asp Glu Glu Val Gln Ser Ser Phe Glu Ser Val Leu Ala Ser
            340                 345                 350

Lys Ser Asp Lys Ser Glu Ile Phe Ser Ser Leu Gly Asp Met Glu Ala
        355                 360                 365

Ser Pro Leu Glu Val Lys Ile Ala Phe Asn Ser Lys Gly Ile Ile Asn
    370                 375                 380

Gln Gly Leu Ile Ser Val Lys Asp Ser Tyr Cys Ser Asn Leu Ile Val
385                 390                 395                 400

Lys Gln Ile Glu Asn Arg Tyr Lys Ile Leu Asn Asn Ser Leu Asn Pro
                405                 410                 415

Ala Ile Ser Glu Asp Asn Asp Phe Asn Thr Thr Thr Asn Thr Phe Ile
            420                 425                 430

Asp Ser Ile Met Ala Glu Ala Asn Ala Asp Asn Gly Arg Phe Met Met
        435                 440                 445

Glu Leu Gly Lys Tyr Leu Arg Val Gly Phe Phe Pro Asp Val Lys Thr
    450                 455                 460

Thr Ile Asn Leu Ser Gly Pro Glu Ala Tyr Ala Ala Ala Tyr Gln Asp
465                 470                 475                 480

Leu Leu Met Phe Lys Glu Gly Ser Met Asn Ile His Leu Ile Glu Ala
                485                 490                 495

Asp Leu Arg Asn Phe Glu Ile Ser Lys Thr Asn Ile Ser Gln Ser Thr
            500                 505                 510

Glu Gln Glu Met Ala Ser Leu Trp Ser Phe Asp Asp Ala Arg Ala Lys
        515                 520                 525

Ala Gln Phe Glu Glu Tyr Lys Arg Asn Tyr Phe Glu Gly Ser Leu Gly
    530                 535                 540

Glu Asp Asp Asn Leu Asp Phe Ser Gln Asn Ile Val Val Asp Lys Glu
545                 550                 555                 560

Tyr Leu Leu Glu Lys Ile Ser Ser Leu Ala Arg Ser Ser Glu Arg Gly
                565                 570                 575

Tyr Ile His Tyr Ile Val Gln Leu Gln Gly Asp Lys Ile Ser Tyr Glu
            580                 585                 590

Ala Ala Cys Asn Leu Phe Ala Lys Thr Pro Tyr Asp Ser Val Leu Phe
        595                 600                 605

Gln Lys Asn Ile Glu Asp Ser Glu Ile Ala Tyr Tyr Tyr Asn Pro Gly
    610                 615                 620

Asp Gly Glu Ile Gln Glu Ile Asp Lys Tyr Lys Ile Pro Ser Ile Ile
625                 630                 635                 640

Ser Asp Arg Pro Lys Ile Lys Leu Thr Phe Ile Gly His Gly Lys Asp
                645                 650                 655

Glu Phe Asn Thr Asp Ile Phe Ala Gly Phe Asp Val Asp Ser Leu Ser
            660                 665                 670

Thr Glu Ile Glu Ala Ala Ile Asp Leu Ala Lys Glu Asp Ile Ser Pro
        675                 680                 685

Lys Ser Ile Glu Ile Asn Leu Leu Gly Cys Asn Met Phe Ser Tyr Ser
    690                 695                 700

Ile Asn Val Glu Glu Thr Tyr Pro Gly Lys Leu Leu Leu Lys Val Lys
705                 710                 715                 720

Asp Lys Ile Ser Glu Leu Met Pro Ser Ile Ser Gln Asp Ser Ile Ile
                725                 730                 735

Val Ser Ala Asn Gln Tyr Glu Val Arg Ile Asn Ser Glu Gly Arg Arg
            740                 745                 750

Glu Leu Leu Asp His Ser Gly Glu Trp Ile Asn Lys Glu Glu Ser Ile
        755                 760                 765

Ile Lys Asp Ile Ser Ser Lys Glu Tyr Ile Ser Phe Asn Pro Lys Glu
    770                 775                 780

Asn Lys Ile Thr Val Lys Ser Lys Asn Leu Pro Glu Leu Ser Thr Leu
785                 790                 795                 800

Leu Gln Glu Ile Arg Asn Asn Ser Asn Ser Ser Asp Ile Glu Leu Glu
                805                 810                 815

Glu Lys Val Met Leu Thr Glu Cys Glu Ile Asn Val Ile Ser Asn Ile
            820                 825                 830

Asp Thr Gln Ile Val Glu Glu Arg Ile Glu Glu Ala Lys Asn Leu Thr
        835                 840                 845

Ser Asp Ser Ile Asn Tyr Ile Lys Asp Glu Phe Lys Leu Ile Glu Ser
    850                 855                 860

Ile Ser Asp Ala Leu Cys Asp Leu Lys Gln Gln Asn Glu Leu Glu Asp
865                 870                 875                 880

Ser His Phe Ile Ser Phe Glu Asp Ile Ser Glu Thr Asp Glu Gly Phe
                885                 890                 895

Ser Ile Arg Phe Ile Asn Lys Glu Thr Gly Glu Ser Ile Phe Val Glu
            900                 905                 910

Thr Glu Lys Thr Ile Phe Ser Glu Tyr Ala Asn His Ile Thr Glu Glu
        915                 920                 925

Ile Ser Lys Ile Lys Gly Thr Ile Phe Asp Thr Val Asn Gly Lys Leu
    930                 935                 940

Val Lys Lys Val Asn Leu Asp Thr Thr His Glu Val Asn Thr Leu Asn

945                 950                 955                 960

Ala Ala Phe Phe Ile Gln Ser Leu Ile Glu Tyr Asn Ser Ser Lys Glu
                965                 970                 975

Ser Leu Ser Asn Leu Ser Val Ala Met Lys Val Gln Val Tyr Ala Gln
            980                 985                 990

Leu Phe Ser Thr Gly Leu Asn Thr  Ile Thr Asp Ala Ala  Lys Val Val
        995                 1000                1005

Glu Leu  Val Ser Thr Ala Leu  Asp Glu Thr Ile Asp  Leu Leu Pro
    1010                1015                1020

Thr Leu  Ser Glu Gly Leu Pro  Ile Ile Ala Thr Ile  Ile Asp Gly
    1025                1030                1035

Val Ser  Leu Gly Ala Ala Ile  Lys Glu Leu Ser Glu  Thr Ser Asp
    1040                1045                1050

Pro Leu  Leu Arg Gln Glu Ile  Glu Ala Lys Ile Gly  Ile Met Ala
    1055                1060                1065

Val Asn  Leu Thr Thr Ala Thr  Thr Ala Ile Ile Thr  Ser Ser Leu
    1070                1075                1080

Gly Ile  Ala Ser Gly Phe Ser  Ile Leu Leu Val Pro  Leu Ala Gly
    1085                1090                1095

Ile Ser  Ala Gly Ile Pro Ser  Leu Val Asn Asn Glu  Leu Val Leu
    1100                1105                1110

Arg Asp  Lys Ala Thr Lys Val  Val Asp Tyr Phe Lys  His Val Ser
    1115                1120                1125

Leu Val  Glu Thr Glu Gly Val  Phe Thr Leu Leu Asp  Asp Lys Ile
    1130                1135                1140

Met Met  Pro Gln Asp Asp Leu  Val Ile Ser Glu Ile  Asp Phe Asn
    1145                1150                1155

Asn Asn  Ser Ile Val Leu Gly  Lys Cys Glu Ile Trp  Arg Met Glu
    1160                1165                1170

Gly Gly  Ser Gly His Thr Val  Thr Asp Asp Ile Asp  His Phe Phe
    1175                1180                1185

Ser Ala Pro Ser Ile Thr Tyr Arg Glu Pro His Leu Ser Ile Tyr
    1190                1195                1200

Asp Val Leu Glu Val Gln Lys Glu Glu Leu Asp Leu Ser Lys Asp
    1205                1210                1215

Leu Met Val Leu Pro Asn Ala Pro Asn Arg Val Phe Ala Trp Glu
    1220                1225                1230

Thr Gly Trp Thr Pro Gly Leu Arg Ser Leu Glu Asn Asp Gly Thr
    1235                1240                1245

Lys Leu Leu Asp Arg Ile Arg Asp Asn Tyr Glu Gly Glu Phe Tyr
    1250                1255                1260

Trp Arg Tyr Phe Ala Phe Ile Ala Asp Ala Leu Ile Thr Thr Leu
    1265                1270                1275

Lys Pro Arg Tyr Glu Asp Thr Asn Ile Arg Ile Asn Leu Asp Ser
    1280                1285                1290

Asn Thr Arg Ser Phe Ile Val Pro Ile Ile Thr Thr Glu Tyr Ile
    1295                1300                1305

Arg Glu Lys Leu Ser Tyr Ser Phe Tyr Gly Ser Gly Gly Thr Tyr
    1310                1315                1320

Ala Leu Ser Leu Ser Gln Tyr Asn Met Gly Ile Asn Ile Glu Leu
    1325                1330                1335

Ser Glu Ser Asp Val Trp Ile Ile Asp Val Asp Asn Val Val Arg
    1340                1345                1350

Asp Val Thr Ile Glu Ser Asp Lys Ile Lys Lys Gly Asp Leu Ile
    1355                1360                1365

Glu Gly Ile Leu Ser Thr Leu Ser Ile Glu Glu Asn Lys Ile Ile
    1370                1375                1380

Leu Asn Ser His Glu Ile Asn Phe Ser Gly Glu Val Asn Gly Ser
    1385                1390                1395

Asn Gly Phe Val Ser Leu Thr Phe Ser Ile Leu Glu Gly Ile Asn
    1400                1405                1410

Ala Ile Ile Glu Val Asp Leu Leu Ser Lys Ser Tyr Lys Leu Leu
    1415                1420                1425

Ile Ser Gly Glu Leu Lys Ile Leu Met Leu Asn Ser Asn His Ile
    1430                1435                1440

Gln Gln Lys Ile Asp Tyr Ile Gly Phe Asn Ser Glu Leu Gln Lys
    1445                1450                1455

Asn Ile Pro Tyr Ser Phe Val Asp Ser Glu Gly Lys Glu Asn Gly
    1460                1465                1470

Phe Ile Asn Gly Ser Thr Lys Glu Gly Leu Phe Val Ser Glu Leu
    1475                1480                1485

Pro Asp Val Val Leu Ile Ser Lys Val Tyr Met Asp Asp Ser Lys
    1490                1495                1500

Pro Ser Phe Gly Tyr Tyr Ser Asn Asn Leu Lys Asp Val Lys Val
    1505                1510                1515

Ile Thr Lys Asp Asn Val Asn Ile Leu Thr Gly Tyr Tyr Leu Lys
    1520                1525                1530

Asp Asp Ile Lys Ile Ser Leu Ser Leu Thr Leu Gln Asp Glu Lys
    1535                1540                1545

Thr Ile Lys Leu Asn Ser Val His Leu Asp Glu Ser Gly Val Ala
    1550                1555                1560

Glu Ile Leu Lys Phe Met Asn Arg Lys Gly Asn Thr Asn Thr Ser
    1565                1570                1575

Asp Ser Leu Met Ser Phe Leu Glu Ser Met Asn Ile Lys Ser Ile
    1580                1585                1590

Phe Val Asn Phe Leu Gln Ser Asn Ile Lys Phe Ile Leu Asp Ala
    1595                1600                1605

Asn Phe Ile Ile Ser Gly Thr Thr Ser Ile Gly Gln Phe Glu Phe
    1610                1615                1620

Ile Cys Asp Glu Asn Asp Asn Ile Gln Pro Tyr Phe Ile Lys Phe
    1625                1630                1635

Asn Thr Leu Glu Thr Asn Tyr Thr Leu Tyr Val Gly Asn Arg Gln
    1640                1645                1650

Asn Met Ile Val Glu Pro Asn Tyr Asp Leu Asp Asp Ser Gly Asp
    1655                1660                1665

Ile Ser Ser Thr Val Ile Asn Phe Ser Gln Lys Tyr Leu Tyr Gly
    1670                1675                1680

Ile Asp Ser Cys Val Asn Lys Val Val Ile Ser Pro Asn Ile Tyr
    1685                1690                1695

Thr Asp Glu Ile Asn Ile Thr Pro Val Tyr Glu Thr Asn Asn Thr
    1700                1705                1710

Tyr Pro Glu Val Ile Val Leu Asp Ala Asn Tyr Ile Asn Glu Lys
    1715                1720                1725

Ile Asn Val Asn Ile Asn Asp Leu Ser Ile Arg Tyr Val Trp Ser
    1730                1735                1740

Asn Asp Gly Asn Asp Phe Ile Leu Met Ser Thr Ser Glu Glu Asn
    1745                1750                1755

Lys Val Ser Gln Val Lys Ile Arg Phe Val Asn Val Phe Lys Asp
    1760                1765                1770

Lys Thr Leu Ala Asn Lys Leu Ser Phe Asn Phe Ser Asp Lys Gln
    1775                1780                1785

Asp Val Pro Val Ser Glu Ile Ile Leu Ser Phe Thr Pro Ser Tyr
    1790                1795                1800

Tyr Glu Asp Gly Leu Ile Gly Tyr Asp Leu Gly Leu Val Ser Leu
    1805                1810                1815

Tyr Asn Glu Lys Phe Tyr Ile Asn Asn Phe Gly Met Met Val Ser
    1820                1825                1830

Gly Leu Ile Tyr Ile Asn Asp Ser Leu Tyr Tyr Phe Lys Pro Pro
    1835                1840                1845

Val Asn Asn Leu Ile Thr Gly Phe Val Thr Val Gly Asp Asp Lys
    1850                1855                1860

Tyr Tyr Phe Asn Pro Ile Asn Gly Gly Ala Ala Ser Ile Gly Glu
    1865                1870                1875

Thr Ile Ile Asp Asp Lys Asn Tyr Tyr Phe Asn Gln Ser Gly Val
    1880                1885                1890

Leu Gln Thr Gly Val Phe Ser Thr Glu Asp Gly Phe Lys Tyr Phe

1895                1900                1905

Ala Pro Ala Asn Thr Leu Asp Glu Asn Leu Glu Gly Glu Ala Ile
    1910                1915                1920

Asp Phe Thr Gly Lys Leu Ile Ile Asp Glu Asn Ile Tyr Tyr Phe
    1925                1930                1935

Asp Asp Asn Tyr Arg Gly Ala Val Glu Trp Lys Glu Leu Asp Gly
    1940                1945                1950

Glu Met His Tyr Phe Ser Pro Glu Thr Gly Lys Ala Phe Lys Gly
    1955                1960                1965

Leu Asn Gln Ile Gly Asp Tyr Lys Tyr Tyr Phe Asn Ser Asp Gly
    1970                1975                1980

Val Met Gln Lys Gly Phe Val Ser Ile Asn Asp Asn Lys His Tyr
    1985                1990                1995

Phe Asp Asp Ser Gly Val Met Lys Val Gly Tyr Thr Glu Ile Asp
    2000                2005                2010

Gly Lys His Phe Tyr Phe Ala Glu Asn Gly Glu Met Gln Ile Gly
    2015                2020                2025

Val Phe Asn Thr Glu Asp Gly Phe Lys Tyr Phe Ala His His Asn
    2030                2035                2040

Glu Asp Leu Gly Asn Glu Glu Gly Glu Glu Ile Ser Tyr Ser Gly
    2045                2050                2055

Ile Leu Asn Phe Asn Asn Lys Ile Tyr Tyr Phe Asp Asp Ser Phe
    2060                2065                2070

Thr Ala Val Val Gly Trp Lys Asp Leu Glu Asp Gly Ser Lys Tyr
    2075                2080                2085

Tyr Phe Asp Glu Asp Thr Ala Glu Ala Tyr Ile Gly Leu Ser Leu
    2090                2095                2100

Ile Asn Asp Gly Gln Tyr Tyr Phe Asn Asp Asp Gly Ile Met Gln
    2105                2110                2115

Val Gly Phe Val Thr Ile Asn Asp Lys Val Phe Tyr Phe Ser Asp
    2120                2125                2130

Ser Gly Ile Ile Glu Ser Gly Val Gln Asn Ile Asp Asp Asn Tyr
    2135                2140                2145

Phe Tyr Ile Asp Asp Asn Gly Ile Val Gln Ile Gly Val Phe Asp
    2150                2155                2160

Thr Ser Asp Gly Tyr Lys Tyr Phe Ala Pro Ala Asn Thr Val Asn
    2165                2170                2175

Asp Asn Ile Tyr Gly Gln Ala Val Glu Tyr Ser Gly Leu Val Arg
    2180                2185                2190

Val Gly Glu Asp Val Tyr Tyr Phe Gly Glu Thr Tyr Thr Ile Glu
    2195                2200                2205

Thr Gly Trp Ile Tyr Asp Met Glu Asn Glu Ser Asp Lys Tyr Tyr
    2210                2215                2220

Phe Asn Pro Glu Thr Lys Lys Ala Cys Lys Gly Ile Asn Leu Ile
    2225                2230                2235

Asp Asp Ile Lys Tyr Tyr Phe Asp Glu Lys Gly Ile Met Arg Thr
    2240                2245                2250

Gly Leu Ile Ser Phe Glu Asn Asn Asn Tyr Tyr Phe Asn Glu Asn
    2255                2260                2265

Gly Glu Met Gln Phe Gly Tyr Ile Asn Ile Glu Asp Lys Met Phe
    2270                2275                2280

Tyr Phe Gly Glu Asp Gly Val Met Gln Ile Gly Val Phe Asn Thr
    2285                2290                2295

Pro Asp Gly Phe Lys Tyr Phe Ala His Gln Asn Thr Leu Asp Glu
    2300                2305                2310

Asn Phe Glu Gly Glu Ser Ile Asn Tyr Thr Gly Trp Leu Asp Leu
    2315                2320                2325

Asp Glu Lys Arg Tyr Tyr Phe Thr Asp Glu Tyr Ile Ala Ala Thr
    2330                2335                2340

Gly Ser Val Ile Ile Asp Gly Glu Glu Tyr Tyr Phe Asp Pro Asp
    2345                2350                2355

Thr Ala Gln Leu Val Ile Ser Glu
    2360                2365